# EUROPEAN PATENT APPLICATION

(11) **EP 2 264 035 A1**
(43) Date of publication of application: **22.12.2010**
(21) Application number: 09007403.0
(22) Date of filing: 04.06.2009
(51) Int. Cl.: C07D 487/04, A61K 31/519, A61P 25/00

(54) **Glycine B antagonists**

(71) Applicant: Merz Pharma GmbH & Co. KGaA, 60318 Frankfurt (DE)
(72) Inventor: The designation of the inventor has not yet been filed
(74) Representative: Goddard, Christopher Robert

(57) **Abstract**

The invention relates to pyrazolopyrimidine derivatives as well as their pharmaceutically acceptable salts. The invention further relates to a process for the preparation of such compounds. The compounds of the invention are glycine B antagonists and are therefore useful for the control and prevention of various disorders, including neurological disorders,

## Description

### FIELD OF THE INVENTION

The present invention relates to novel pyrazolopyrimidine derivatives which may act as glycine B antagonists, methods for their synthesis and the treatment and/or prevention of various diseases and disorders, including neurological disorders, by administration of such substances.

### BACKGROUND OF THE INVENTION

Glutamate is a major excitatory transmitter in the central nervous system and is believed to be involved in many pathological and excitotoxic processes; therefore, there is a great deal of interest in the development of glutamate antagonists for therapeutic uses. Glutamate activates three major types of ionotropic receptors: α-amino-3-hydroxy-5-methyl-4-isoazolepropionic acid (AMPA), kainate, and N-methyl-D-aspartate (NMDA) as well as several types of metabotropic receptors. Antagonism of NMDA receptors potentially has a wide range of therapeutic applications. Functional inhibition of NMDA receptors may be achieved through actions at different recognition sites, such as the primary transmitter site, the strychnine insensitive glycine site (glycine B), the polyamine site, and the phencyclidine site located inside the cation channel.

Receptor desensitization may represent a physiological process serving as an endogenous control mechanism to prevent long term neurotoxic activation of glutamate receptors but allow their transient physiological activation. In the case of the NMDA receptor, the co-agonist glycine is an endogenous ligand inhibiting such desensitization via activation of the glycine B site. It is noteworthy that ischemia increases not only the concentration of extracellular glutamate but also that of glycine and, although this latter effect is less pronounced, it actually persists for a longer period of time. Thus, glycine B antagonists may restore normal synaptic transmission under such conditions by increasing NMDA receptor desensitization to its physiological level. It has been suggested that glycine B antagonists may offer a better therapeutic window than agents acting at other recognition sites of the NMDA receptor complex.

Therefore, glycine B antagonists, such as glycine B antagonists restricted to action in the peripheral nervous system (PNS), may be useful for the treatment and/or prevention of pain, including acute pain, chronic pain, allodynia, hyperalgesia, visceral pain, phantom pain, post-operative pain, neuropathic pain, peripheral neuropathy including, for example peripheral neuropathy induced by nociception, inflammation, ischemia, viral infection (HZV), traumatic and other mechanical nerve injury, cancer, diabetes mellitus, HIV infection, fibromyalgia, trigeminus neuralgia, inflammatory bowel diseases (IBD), irritative bowel syndrome (IBS), arthritis including rheumatoid arthritis, osteoarthritis (degenerative joint disease), multiple sclerosis (MS) and gout (metabolic arthritis).

Glycine B antagonists may also be useful for the treatment and/or prevention of acute insults, including cerebral ischemia, cerebral infarct, brain oedema, anoxia, inner ear insult, inner ear insult in tinnitus, head or brain or spinal cord trauma, head or brain or spinal cord injuries, trauma, sound- or drug-induced inner ear insult, ischaemia resulting from cardiac arrest or stroke or bypass operations or transplants, acute pain, hypoxia, perinatal hypoxia, and ischaemia;
chronic insults, such as neurodegenerative disorders, including Morbus Huntington, Alzheimer's disease Creutzfeld-Jakob's syndrome/disease, bovine spongiform encephalopathy (BSE) prion related infections, diseases involving mitochondrial dysfunction, diseases involving β-amyloid and/or tauopathy, Down's syndrome, motor neuron diseases, amyotrophic lateral sclerosis (ALS), olivoponto-cerebellar atrophy, Parkinson's disease, Neuronal Ceroid Lipofuscinosis, AIDS dementia complex, AIDS-related dementia, dementia related to HIV infections, HIV-1 encephalopathy, AIDS encephalopathy, Korsakoff syndrome, vascular dementia, and corticobasal degeneration;
neurological disorders, including tinnitus, hearing loss, sound- or drug-induced tinnitus, haloperidol-induced dyskinesias, dopaminomimetic-induced dyskinesias, chorea, Huntington's chorea, athetosis, dystonia, stereotypy, ballism, tardive dyskinesias, tic disorder, spasmodic torticollis, blepharospasm, focal and generalized dystonia, nystagmus, Parkinson's dementia, mild cognitive impairment, cognitive deficits in various forms of mild cognitive impairment, cognitive deficits in various forms of dementia, dementia pugilistica, vascular and frontal lobe dementia, cognitive impairment, learning impairment, L-dopa-induced dykinesias, L-dopa-induced dykinesias in Parkinson's disease therapy, dyskinesias, dyskinesia in Huntington's disease, drug induced dykinesias, neuroleptic-induced dyskinesias, neurodegenerative cerebellar ataxias, centrally induced neuropathic pain, convulsions, epileptic convulsions, epilepsy, temporal lobe epilepsy, myoclonic epilepsy, tremor, dementia in Alzheimer's disease, dementia in Korsakoff syndrome, dementia, hereditary cerebellar ataxias, sleep disorders, movement disorders, essential tremor, muscle spasms, and spasticity;
psychological/psychiatric disorders, including generalized anxiety disorder, obsessive-compulsive disorder, panic disorder, posttraumatic stress disorder, social phobia, phobic disorders, substance-induced anxiety disorder, delusional disorder, schizoaffective disorder, schizophreniform disorder, substance-induced psychotic disorder, delirium, post-operative cognitive deficit (POCD), cognitive impairment, learning impairment, anxiety disorders, panic disorders, anxiety and panic disorders, social anxiety disorder (SAD), attention deficit hyperactivity disorder (ADHD), attention deficit syndrome (ADS), dementia, posttraumatic stress disorder (PTSD), schizophrenia, positive or cognitive or negative symptoms of schizophrenia, major depressive disorder, major depression, depression, bipolar manic-depressive disorder, sleep disorders, agoraphobia, bulimia nervosa, eating disorders, obesity, obesity-related disorders, obesity abuse, food addiction, binge eating disorders, and hyperactivity in children;
drug/alcohol abuse, including craving (e.g., for drugs of abuse), abuse, addiction, nicotine addiction, nicotine abuse, alcohol addiction, alcohol abuse, opiate addiction, opiate abuse, cocaine addiction, cocaine abuse, amphetamine addiction, and amphetamine abuse;
skin diseases, including atopic dermatitis, itching, skin lesions induced by severe itching or atopic dermatitis, systemic sclerosis, pruritic conditions, and pruritis;
diseases of the gastro-intestinal tract and metabolic diseases, including diarrhoea, hepatic encephalopathy, hypoglycaemia, gastroesophageal reflux disease (GERD), gastrointestinal dysfunction, lower esophageal sphincter (LES) disease, functional gastrointestinal disorders, dyspepsia, vomiting, urinary incontinence, and regurgitation;
diseases of the immune system, including Sjogren's syndrome, systemic lupus erythematosus, and multiple sclerosis (MS);
eye diseases, including eye injuries, eye diseases, eye disorders, glaucoma, retinopathy, and macular degeneration;
diseases of the respiratory tract, including respiratory tract infection, chronic laryngitis, asthma, reflux-related asthma, and lung disease;
migraine; autism; restless leg syndrome (RLS); Tourette syndrome; micturition disorders; neuromuscular disorder in the lower urinary tract; and drug tolerance to opioids.

A number of pyrazolopyrimidine derivatives, including pyrazolo[1,5-a]pyrimidine-2,7-dione derivatives, have been previously described. Pyrazolo[1,5-a]pyrimidine-2,7-dione derivatives have generally been described in different tautomeric forms, i.e., as pyrazolo[1,5-a]pyrimidine-2,7-diol derivatives.

International Publication No. WO 2008/062026 discloses pyrazolo[1,5-a]pyrimidinol derivatives of general formula (I) as chemokine CXCR2 antagonists: 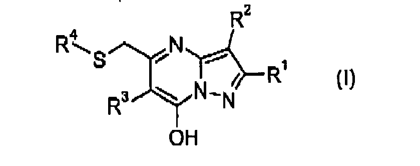 Including tautomers which may be represented by following forms: 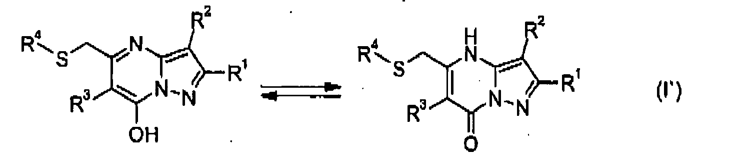 wherein R¹, R², and R³ independently are H, CN, alkyl, alkoxy, (un)substituted aryl, etc.; or one of R¹ or R² is oxo and the other is H; or R¹ and R² together form an (un)substituted alicyclic or aromatic ring optionally containing at least one heteroatom selected from N, O, or S and is optionally annelated with an alicyclic or aromatic ring; and R⁴ may be (un)substituted aryl or heterocyclyl. 5-(2,3-difluorophenylsulfanylmethyl)-pyrazolo[1,5-a]pyrimidine-2,7-diol is disclosed as an example:

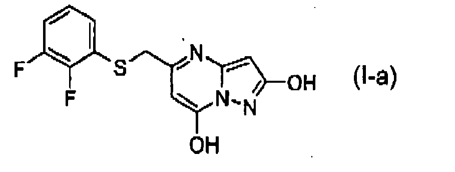

3-(1,3,4-Thiadiazol-2-yl)pyrazolo[1,5-a]pyrimidine derivatives (3) were studied as potential antimicrobial agents [Auzzi, G; Costanzo, A; Bruni, F; Clauser, M; Guerrini, G; Selleri, S; Vettori, L. P. Farmaco 1990, 45(11), 1193-205.]: 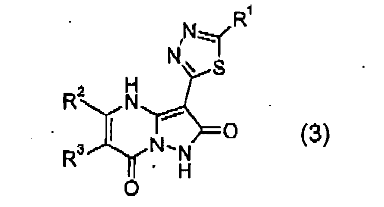 wherein R¹ = H, Me, Ph; R² = H, CO₂Et; R³ = H, Me.

Syntheses of pyrazolo[1,5- a]pyrimidines (4) and their reactions with phenyldiazonium salts to give 5-phenylazo-pyrazolo[1,5-a]pydmidines (5) has been reported [Ried, W.; Peuchert, K. P. Justus Liebigs Ann. Chem. 1962, 660, 104-117.] 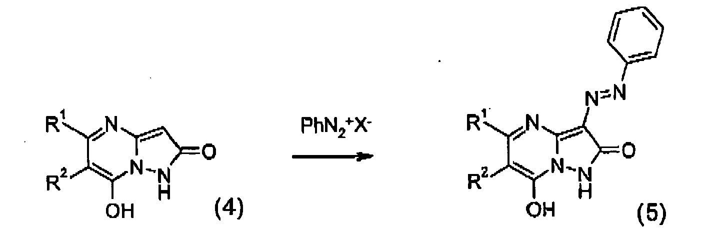 wherein R¹ = Me; R² = H, Me, Et.

The reaction of 4-arylazo-3-aminopyrazol-5-ones (6) with active methylene reagents and nitrile imines was used to prepare (7) (analogs of (5)) [Elgemeie, G. H.; Elghandour, A. H.; Elshimy, H. M. J.Prakt Chem. 1989, 331 (3), 466-474.]: 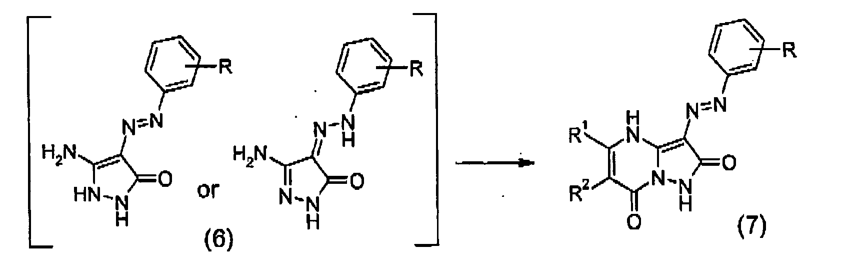 wherein R = Cl, MeO; R¹ = H, Me; R² = H, CN.

Cyclocondensation of 3-amino-3-pyrazolin-5-one (8) with RCOCH₂COOEt (9) was reported to give 5-substituted pyrazolo[1,5-a]pyrimidines (10) [Balicki, R; Kaczmarek, L; Nantka-Namirski, P. Polish J. Chem. 1979, 53(12), 2491-2499. and Balicki, R. Polish J. Chem. 1983, 57(10-12), 1251-1261.]: 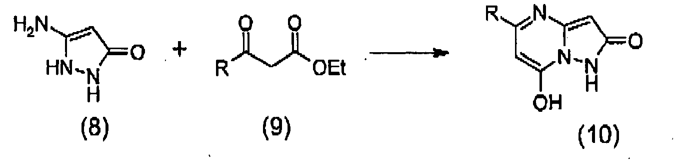 wherein R = Me, Ph, 3-Pyridyl, 4-Pyridyl.

A similar cyclocondensation procedure employing propyl 5-amino-3-oxo-2,3-dihydro-1H-pyrazole-4-carboxylate was shown to give 3-carboalkoxy-derivative (11) of pyrazolo[1,5-a]pyrimidine (10) [Gavrilenko, B. B. Zhurnal Organicheskoi Khimii (Russian) 1982, 18(5), 1079-1084.]

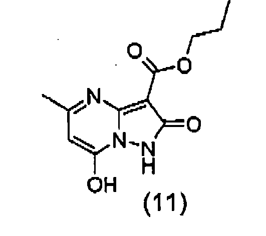

### THE PRESENT INVENTION

We have determined that certain pyrazolopyrimidine derivatives are glycine B antagonists. Therefore, these substances may be therapeutically beneficial in the treatment of conditions which involve excitotoxicity and malfunctioning of glutamatergic neurotransmission. These substances may be administered in the form of a pharmaceutical composition, wherein they are present together with one or more pharmaceutically acceptable diluents, carriers, or excipients.

### OBJECTS OF THE INVENTION

It is an object of the present invention to provide novel pharmaceutical compounds which are glycine B antagonists and pharmaceutical compositions thereof. It is a further object of the invention to provide a novel method of treating, eliminating, alleviating, palliating, or ameliorating undesirable conditions, including CNS conditions, associated with excitotoxicity and malfunctioning of glutamatergic neurotransmission by employing a compound of the invention or a pharmaceutical composition containing the same.

An additional object of the invention is the provision of processes for producing the pyrazolopyrimidine derivatives.

Yet additional objects will become apparent hereinafter, and still further objects will be apparent to one skilled in the art.

### SUMMARY OF THE INVENTION

What we therefore believe to be comprised by our invention may be summarized inter alia in the following words:
A compound selected from those of Formula I: 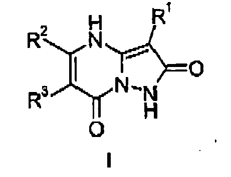 wherein
   R¹ represents hydrogen, trifluoromethyl, C₁₋₆alkyl, COOH, Calkoxycarbonyl, hydroxy-C₁₋₆alkoxycarbonyl, aryl-C₁₋₆alkoxycarbonyl, heteroaryl-C₁₋₆alkoxycarbonyl, aryl, heteroaryl, aryl-C₁₋₆alkyl, heteroaryl-C₁₋₆alkyl, cyclo-C₅₋₁₂alkyl-C₁₋₆alkyl, aryl-C₂₋₆alkenyl, heteroaryl-C₂₋₆alkenyl, arylamino, heteroarylamino, aryl-C₁₋₆alkylamino; heteroaryl-C₁₋₆alkylamino; acylamino, arylsulfonylamino, C₁₋₆alkylaminocarbonyl, cyclo-C₃₋₁₂alkylaminocarbonyl, arylaminocarbonyl, heteroarylaminocarbonyl, aryi-C₁₋₆alkylaminocarbonyl, heteroaryl-C₁₋₆alkylaminocarbonyl, hydroxy-C₁₋₆alkylaminocarbonyl, di-(C₁₋₆alkyl)aminocarbonyl, aryl(C₁₋₆alkyl)aminocarbonyl, heteroaryl(C₁₋₆alkyl)aminocarbonyl, hydroxy-C₁₋₆alkyl, C₁₋₆alkoxy-C₁₋₆alkyl, carboxy-C₁₋₆alkyl, carbamoyl-C₁₋₆alkyl, amino-C₁₋₆alkyl, acylamino-C₁₋₆alkyl, arylamino-C₁₋₆alkyl, aryloxy-C₁₋₆alkyl, C₁₋₆alkylsulfonylamino-C₁₋₆alkyl, arylsulfonylamino-Cvalkyl, C₁₋₆₋alkylaminocarbonyl-C₁₋₆alkyl, cyclo-C₃₋₁₂alkylaminocarbonyl-C₁₋₆alkyl, arylaminocarbonyl-C₁₋₆alkyl, heteroarylaminocarbonyl-C₁₋₆alkyl, aryl-C₁₋₆alkylaminocarbonyl-C₁₋₆alkyl, heteroaryl-C₁₋₆alkylaminocarbonyl-C₁₋₆alkyl, hydroxy-C₁₋₆alkylaminocarbonyl-C₁₋₆alkyl, carboxy-C₁₋₆alkylaminocarbonyl-C₁₋₆alkyl, di-(C₁₋₆alkyl)aminocarbonyl-C₁₋₆alkyl, aryl(C₁₋₆alkyl)aminocarbonyl-C₁₋₆alkyl, heteroaryl(C₁₋₆alkyl)aminocarbonyl-C₁₋₆alkyl, aryl-heterocyclyl-C₁₋₆alkyl, or heteroaryl-heterocyclyl-C₁₋₆alkyl; and
   R² and R³ together represent -(CH₂)ₙ- wherein n is 3, 4, or 5;
   or R² and R³ together with the C=C bond to which they are attached represent a phenyl ring or a 5-, 6- or 7-membered ring which may be unsaturated, or partly unsaturated, and wherein the ring in addition to nitrogen atom may contain heteroatom selected from sulfur, oxygen and nitrogen and may be substituted by one or more substituents selected from C₁-₆alkyl and halogen;
   wherein
   the term "aryl" represents phenyl or naphthyl, or phenyl substituted by one or more substituents selected independently from halogen, amino, hydroxy, nitro, cyano, COOH, trifluoromethyl, C₁₋₆alkyl. C₂₋₆alkenyl, C₂₋₆alkynyl, heteroaryl, C₁₋₆alkoxy, difluoromethoxy, trifluoromethoxy, cyclo-C_{3- 12}alkoxy, aryloxy, heteroaryloxy, aryl-C₁₋₆alkoxy, heteroaryl-C₁₋₆alkoxy, amino-C₁₋₆alkyl, hydroxy-C₁₋₆alkyl, carboxy-C₁₋₆alkyl, carbamoyl-C₁₋₆alkyl, C₁₋₆alkoxy-C₁₋₆alkyl, hydroxy-C₁₋₆alkoxy, C₁₋₆alkoxy-C₁₋₆alkoxy, carboxy-C₁₋₆alkoxy, C₁₋₆alkoxycarbonyl, C₁₋₆alkylamino, cyclo-C₃₋₁₂alkylamino, arylamino, heteroarylamino, aryl-C₁₋₆alkylamino, heteroaryl-C₁₋₆alkylamino, hydroxy-C₁₋₆alkylamino, carboxy-C₁₋₆alkylamino, C₁₋₆alkylamino-C₁₋₆alkyl, di-(C₁₋₆alkyl)amino, acylamino, di-(C₁₋₆alkyl)amino-C₁₋₆alkyl, carboxy-C₁₋₆alkylamino-C₁₋₆alkyl, C₁₋₆alkylamino-C₁₋₆alkoxy, di-(C₁₋₆alkyl)amino-C₁₋₆alkoxy carboxy-C₁₋₆alkylamino-C₁₋₆alkoxy, C₁₋₆alkylsulfonylamino, arylsulfonylamino, C₁₋₆alkylsulfonylamino-C₁₋₆alkyl, C₁₋₆alkyl-aminosulfonyl, di-(C₁₋₆alkyl)aminosulfonyl, arylaminosulfonyl, heteroarylaminosulfonyl, C₁₋₆alkylaminocarbonyl-C₁₋₆alkyl, cyclo-C₃₋₁₂alkylaminocarbonyl-C₁₋₆alkyl, arylaminocarbonyl-C₁₋₆alkyl, heteroarylaminocarbonyl-C₁₋₆alkyl, aryl-C₁₋₆alkylaminocarbonyl-C₁₋₆alkyl, heteroaryl-C₁₋₆alkylaminocarbonyl-C₁₋₆alkyl, hydroxy-C₁₋₆alkylaminocarbonyl-C₁₋₆alkyl, carboxy-C₁₋₆alkylaminocarbonyl-C₁₋₆alkyl, di-(C₁₋₆alkyl)aminocarbonyl-C₁₋₆alkyl, aryl(C₁₋₆alkyl)aminocarbonyl-C₁₋₆alkyl, and heteroaryl(C₁₋₆alkyl)aminocarbonyl-C₁₋₆alkyl;
   the term "heteroaryl" represents an aromatic 5-6 membered ring comprising one to four heteroatoms selected from oxygen, sulfur and nitrogen, or a bicyclic group containing a 5-6 membered ring comprising one to four heteroatoms selected from oxygen, sulfur and nitrogen fused with a benzene ring or with a 5-6 membered ring comprising one to four heteroatoms selected from oxygen, sulfur and nitrogen,
   wherein the heteroaryl is optionally substituted by one or more substituents selected independently from halogen, amino, hydroxy, nitro, cyano, COOH, trifluoromethyl, C₁₋₆alkyl, C₂₋₆alkenyl, C₂₋₆alkynyl, heteroaryl, C₁₋₆alkoxy, difluoromethoxy, trifluoromethoxy, cyclo-C₃₋₁₂alkoxy, aryloxy, heteroaryloxy, aryl-C₁₋₆alkoxy, heteroaryl-C₁₋₆alkoxy, amino-C₁₋₆alkyl, hydroxy-C₁₋₆alkyl, carboxy-C₁₋₆alkyl, carbamoylC₁₋₆alkyl, C₁₋₆alkoxy-C₁₋₆alkyl, hydroxy-C₁₋₆alkoxy, C₁₋₆alkoxy-C₁₋₆alkoxy, carboxy-C₁₋₆alkoxy, C₁₋₆alkoxycarbonyl, C₁₋₆alkylamino, cyclo-C₃₋₁₂alkylamino, arylamino, heteroarylamino, aryl-C₁₋₆alkylamino, heteroaryl-C₁₋₆alkylamino, hydroxy-C₁₋₆alkylamino, carboxy-C₁₋₆alkylamino, C₁₋₆alkylamino-C₁₋₆alkyl, di-(C₁₋₆alkyl)amino, acylamino, di-(C₁₋₆alkyl)amino-C₁₋₆alkyl, carboxy-C₁₋₆alkylamino-C₁₋₆lkyl, C₁₋₆alkylamino-C₁₋₆alkoxy, di-(C₁₋₆alkyl)amino-C₁₋₆alkoxy, carboxy-C₁₋₆alkylamino-C₁₋₆alkoxy, C₁₋₆alkylsulfonylamino, arylsulfonylamino, C₁₋₆alkylsulfonylamino-C₁₋₆alkyl, _{C1-6}alkyl-aminosulfonyl, di-(C₁₋₆alkyl)aminosulfonyl, arylaminosulfonyl, heteroarylaminosulfonyl, C₁₋₆alkylaminocarbonyl-C₁₋₆alkyl, cyclo-C₃₋₁₂alkylaminocarbonyl-C₁₋₆alkyl, arylaminocarbonyl-C₁₋₆alkyl, heteroarylaminocarbonyl-C₁₋₆alkyl, aryl-C₁₋₆alkylaminocarbonyl-C₁₋₆alkyl, heteroaryl-C₁₋₆alkylaminocaroonyl-C₁₋₆alkyl, hydmxy-C₁₋₆alkylaminocarbonyl-C₁₋₆alkyl, carboxy-C₁₋₆alkylaminocarbonyl-C_{1- 6}alkyl di-(C₁₋₆alkyl)aminocarbonyl-C₁₋₆alkyl, aryl(C₁₋₆alkyl)aminocarbonyl-C₁₋₆alkyl, heteroaryl(C₁₋₆alkyl)aminocarbonyl-C₁₋₆alkyl;
   its optical isomers, polymorphs, analogs, derivatives, prodrugs, and pharmaceutically-acceptable acid and base addition salts and hydrates and solvates thereof;
   it being understood that:
   if R² and R³ together represent -(CH₂)ₙ-, then R¹ does not represent aryl-C₁₋₆alkyl or hoteroaryl-C₁₋₆alkyl.

Such a compound of Formula I, wherein R¹ represents hydrogen, aryl, arylaminocarbonyl, heteroarylaminocarbonyl, arylC₁₋₆alkylamino, heteroarylC₁₋₆alkylamino, acylamino, arylsulfonylamino, C₁₋₆alkoxycarbonyl, aryl-C₁₋₆alkylaminocarbonyl, aryiC₂₋₆alkenyl, arylC₁₋₆alkoxycarbonyl, heteroarylC₁₋₆alkoxycarbonyl, aryl(C₁₋₆alkyl)aminocarbonyl, heteroaryl(C₁₋₆alkyl)aminocarbonyl, aryl-heterocyclyl-C₁₋₆alkyl, or heteroaryl-heterocyclyl-C₁₋₆alkyl.

Such a compound of Formula I, wherein R¹ represents arylcarbonylamino or heteroarylcarbonylamino.

Such a compound of Formula I, wherein R¹ represents arylaminocarbonyl, heteroarylaminocarbonyl, arylC₁₋₆alkylamino, heteroarylC₁₋₆alkylamino, arylcarbonylamino, heteroarylcarbonylamino, arylsulfonylamino, arylC₁₋₆alkoxycarbonyl, heteroarylC₁₋₆alkoxycarbonyl, aryl(C₁₋₆alkyl)aminocarbonyl, heteroaryl(C₁₋₆alkyl)aminocarbonyl, aryl-heterocyclyl-C₁₋₆alkyl, or heteroaryl-heterocyclyl-C₁₋₆alkyl, wherein the aryl or heteroaryl moeity is optionally substituted by one or more substituents selected from halogen, hydroxy, trifluoromethyl, C₁₋₆alkyl, and C₁₋₆alkoxy.

Such a compound of Formula I, wherein R¹ represents aryi-C₁₋₆alkylamino or heteroaryl-C₁₋₆alkylamino wherein the C₁₋₆alkyl moiety is substituted by 1 or more carboxy groups.

Such a compound of Formula I, wherein wherein R² and R³ together represent - (CH₂)ₙ- wherein n is 3, 4, or 5 or R² and R³ together with the C=C bond to which they are attached represent a phenyl ring.

Such a compound of Formula **I**, wherein R² and R³ together represent - (CH₂)ₙ- wherein n is 4.

Specific compounds of Formula **I** within the present invention include but are not limited to:
5,6,7,8-Tetrahydro-1H,4H-pyrazolo[5,1-b]quinazoline-2,9-dione,
3-Phenyl-5,6,7,8-tetrahydro-1H,4H-pyrazolo[5,1-b]quinazoline-2,9-dione,
2,9-Dioxo-1,2,4,5,6,7,8,9-octahydro-pyrazolo[5,1-b]quinazoline-3-carboxylic acid phenylamide,
N-(2,9-Dioxo-1,2,4,5,6,7,8,9-octahydro-pyrazolo[5,1-b]quinazolin-3-y-benzamide, Ethyl 2,9-dioxo-1,2,4,5,6,7,8,9-octahydro-pyrazolo[5,1-b]quinazoline-3-carboxylate,
2,9-Dloxo-1,2,4,5,6,7,8,9-octahydro-pyrazolo[5,1-b]quinazoline-3-carboxylicacid (4-chloro-phenyl)-amide,
2,9-Dioxo-1,2,4,5,6,7,8,9-octahydro-pyrazolo[5,1-b]quinazoline-3-carboxylic acid (3-chlom-phenyl)-amide,
2,9-Dioxo-1,2,4,5,6,7,8,9-octahydro-pyrazolo[5,1-b]quinazoline-3-carboxylic acid (2-chloro-phenyl)-amide,
2,9-Dioxo-1,2,4,5,6,7,8,9-octahydro-pyrazolo[5,1-b]quinazoline-3-carboxylic acid benzylamide.
3-((E)-Styryl)-5,6,7,8-tetrahydro-1H,4H-pyrazolo[5,1-b]quinazoline-2,9-dione,
3-[(E)-2-(4-Chloro-phenyl)-vinyl]-5,6,7,8-tetrahydro-1H,4H-pyrazolo[5,1-b]quinazoline-2,9-dione,
3-[(E)-2-(3-Chloro-phenyl)-vinyl]-5,6,7,8-tetrahydro-1H,4H-pyrazolo[5,1-b]quinazoline-2,9-dione,
3-[(2,9-Dioxo-1,2,4,5,6,7,8,9-octahydro-pyrazo)o[5,1-b]quinazoline-3-carbonyl)-amino]-benzoic acid,
Benzyl 2,9-dioxo-1,2,4,5,6,7,8,9-octahydro-pyrazolo[5,1-b]quinazoline-3-carboxylate,
2,9-Dioxo-1,2,4,5,6,7,8,9-octahydro-pyrazolo[5,1-b]quinazoline-3-carboxylic acid (4-methoxy-phenyl)-amide,
2,9-Dioxo-1,2,4,5,6,7,8,9-octahydro-pyrazolo[5,1-b]quinazoline-3-carboxylic acid pyridin-3-ylamide,
N-(2,9-Dioxo-1,2,4,9-tetrahydro-pyrazolo[5,1-b]quinazolin-3-yl)-benzamide,
N-(2,9-Dioxo-1,2,4,9-tetrahydro-pyrazolo[5,1-b]quinazolin-3-yl)-4-methoxybenzamide,
N-(2,9-Dioxo-1,2,4,9-tetrahydro-pyrazolo[5,1-b]quinazolin-3-yl)2-fluoro-benzamide,
N-(2,9-Dioxo-1,2,4,9-tetrahydro- pyrazolo[5,1-b]quinazolin-3-yl)-3-chloro-benzamide,
2,9-Dioxo-1,2,4,5,6,7,8,9-octahydro-pyrazolo[5,1-b]quinazoline-3-carboxylic acid pyridin-2-ylamide,
2,9-Dioxo-1,2,4,5,6,7,8,9-octahydro-pyrazolo[5,1-b]quinazoline-3-carboxylic acid pyridin-4-yiamide,
2,9-Dioxo-1,2,4,5,6,7,8,9-octahydro-pyrazolo[5,1-b]quinazoline-3-carboxylic acid furan-3-ylamide,
2,9-Dioxo-1,2,4,5,6,7,8,9-octahydro-pyrazolo[5,1-b]quinazoline-3-carboxylic acid (1H-pyrrol-2-yl)-amide,
2,9-Dioxo-1,2,4,5,6,7,8,9-octahydro-pyrazolo[5,1-b]quinazoline-3-carboxylic acid (3-hydroxy-phenyl)-amide,
2,9-Dioxo-1,2.4,5,6,7,8,9-octahydro-pyrazolo[5,1-b]quinazoline-3-carboxylic acid (4-hydroxy-phenyl)-amide,
2,9-Dioxo-1,2,4,5,6,7,8,9-octahydro-pyrazolo[5,1-b]quinazoline-3-carboxylic acid (2-fluoro-phenyl)-amide,
2,9-Dioxo-1,2,4,5,6,7,8,9-octahydro-pyrazolo[5,1-b]quinazoline-3-carboxylic acid (2-hydroxy-phenyl)amide,
N-(2,9-Dioxo-1,2,4,5,6,7,8,9-octahydro-pyrazolo[5,1-b]quinazolin-3-yl)-2-fluoro-benzamide,
N-(2,9-Dioxo-1,2,4,5,6,7,8,9-octahydro-pyrazolo[5,1-b]quinazolin-3-yl)-2-hydroxybenzamide,
3-[(E)-2-(2-Fluoro-phenyl)-vinyl]-5,6,7,8-tetrahydro-1H,4H-pyrazolo[5,1-b]quinazoline-2,9-dione,
2,9-Dioxo-1,2,4,9-tetrahydro-pyrazolo[5,1-b]quinazoline-3-carboxylic acid,
2,9-Dioxo-1,2,4,9-tetrahydro-pyrazolo[5,1-b]quinazoline-3-carboxylic acid phenylamide,
2,9-Dioxo-1,2,4,9-tetrahydro-pyrazolo[5,1-b]quinazoline-3-carboxylic acid (4-methoxy-phenyl)-amide,
2,9-Dioxo-1,2,4,9-tetrahydro-pyrazolo[5,1-b]quinazoline-3-carboxylic acid (2-fluoro-phenyl)-amide,
2,9-Dioxo-1,2,4,9-tetrahydro-pyrazolo[5,11-b]quinazoline-3-carboxylic acid (3-chloro-phenyl)-amide,
3-(4-Fluoro-phenylamino)-5,6,7,8-tetrahydro-1H,4H-pyrazolo[5,1-b]quinazoline-2,9-dione,
3-(4-Fluoro-phenylamino)-4H-pyrazolo[5,1-b]quinazoline-2,9-dione,
N-(2,9-Dioxo-1,2.4,5,6,7,8,9-octahydro-pyrazolo[5,1-b]quinazolin-3-yl)-4-methyl-benzenesulfonamide,
N-2,9-Dioxo-1,2,4,9-tetrahydro- pyrazolo[5,1-b]quinazolin-3-yl)-4-methyl-benzenesulfonamide,
2-(2,9-Dioxo-1,2,4,5,6,7,8,9-octahydro-pyrazolo[5,1-b]quinazolin-3-ylaminoy3-phenyl-propionic acid,
2-(2,9-Dioxo-1,2,4.9-tetrahydro-pyrazolo[5,1-b]quinazotin-3-ylamino)-3-phenyl-propionic acid,
optical isomers, polymorphs, analogs, derivatives, prodrugs, and pharmaceutically-acceptable acid and base addition salts, hydrates, and solvates thereof.

In a further embodiment the invention relates to a compound of Formula **I** as defined above, or an optical **I** isomer, polymorph, analog, derivative, prodrug, pharmaceutically-acceptable acid or base addition salt, hydrate, or solvate thereof for use in the treatment or prevention of NMDA excitotoxicity or malfunctioning glutamatergic neruotransmission.

Moreover, the invention relates to a compound of Formula I as defined above, or an optical isomer, polymorph, analog, derivative, prodrug, pharmaceutically-acceptable acid or base addition salt, hydrate, or solvate thereof for the treatment or prevention of a condition associated with excitotoxicity and malfunctioning of glutamatergic neurotransmission, including for the conditions selected from those described earlier in the description.

Such conditions include pain, including acute pain, chronic pain, allodynia, hyperalgesia, visceral pain, phantom pain, post-operative pain, neuropathic pain, peripheral neuropathy including, for example peripheral neuropathy induced by nociception, inflammation, ischemia, viral infection (HZV), traumatic and other mechanical nerve injury, cancer, diabetes mellitus, HIV infection, fibromyalgia, trigeminus neuralgia, inflammatory bowel diseases (IBD), irritative bowel syndrome (IBS), arthritis including rheumatoid arthritis, osteoarthritis (degenerative joint disease), multiple sclerosis (MS) and gout (metabolic arthritis).

Such conditions also include acute insults, including cerebral ischemia, cerebral infarct, brain oedema, anoxia, inner ear insult, inner ear insult in tinnitus, head or brain or spinal cord trauma, head or brain or spinal cord injuries, trauma, sound- or drug-induced inner ear insult, ischaemia resulting from cardiac arrest or stroke or bypass operations or transplants, acute pain, hypoxia, perinatal hypoxia, and ischaemia; chronic insults, such as neurodegenerative disorders, including Morbus Huntington, Alzheimer's disease Creutzfeld-Jakob's syndrome/disease, bovine spongiform encephalopathy (BSE) prion related infections, diseases involving mitochondrial dysfunction, diseases involving β-amyloid and/or tauopathy, Down's syndrome, motor neuron diseases, amyotrophic lateral sclerosis (ALS), olivoponto-cerebellar atrophy, Parkinson's disease, Neuronal Ceroid Lipofuscinosis, AIDS dementia complex, AIDS-related dementia, dementia related to HIV infections, HIV-1 encephalopathy, AIDS encephalopathy, Korsakoff syndrome, vascular dementia, and corticobasal degeneration;
neurological disorders, including tinnitus, hearing loss, sound- or drug-induced tinnitus, haloperidol-induced dyskinesias, dopaminomimetic-induced dyskinesias, chorea, Huntington's chorea, athetosis, dystonia, stereotypy, ballism, tardive dyskinesias, tic disorder, spasmodic torticollis, blepharospasm, focal and generalized dystonia, nystagmus, Parkinson's dementia, mild cognitive impairment, cognitive deficits in various forms of mild cognitive impairment, cognitive deficits in various forms of dementia, dementia pugilistica, vascular and frontal lobe dementia, cognitive impairment, learning impairment, L-dopa-induced dykinesias, L-dopa-induced dykinesias in Parkinson's disease therapy, dyskinesias, dyskinesia in Huntington's disease, drug induced dyskinesias, neuroleptic-induced dyskinesias, neurodegenerative cerebellar ataxias, centrally induced neuropathic pain, convulsions, epileptic convulsions, epilepsy, temporal lobe epilepsy, myoclonic epilepsy, tremor, dementia in Alzheimer's disease, dementia in Korsakoff syndrome, dementia, hereditary cerebellar ataxias, sleep disorders, movement disorders, essential tremor, muscle spasms, and spasticity;
psychological/psychiatric disorders, including generalized anxiety disorder, obsessive-compulsive disorder, panic disorder, posttraumatic stress disorder, social phobia, phobic disorders, substance-induced anxiety disorder, delusional disorder, schizoaffective disorder, schizophreniform disorder, substance-induced psychotic disorder, delirium, post-operative cognitive deficit (POCD), cognitive impairment, learning impairment, anxiety disorders, panic disorders, anxiety and panic disorders, social anxiety disorder (SAD), attention deficit hyperactivity disorder (ADHD), attention deficit syndrome (ADS), dementia, posttraumatic stress disorder (PTSD), schizophrenia, positive or cognitive or negative symptoms of schizophrenia, major depressive disorder, major depression, depression, bipolar manic-depressive disorder, sleep disorders, agoraphobia, bulimia nervosa, eating disorders, obesity, obesity-related disorders, obesity abuse, food addiction, binge eating disorders, and hyperactivity in children;
drug/alcohol abuse, including craving (e.g., for drugs of abuse), abuse, addiction, nicotine addiction, nicotine abuse, alcohol addiction, alcohol abuse, opiate addiction, opiate abuse, cocaine addiction, cocaine abuse, amphetamine addiction, and amphetamine abuse;
skin diseases, including atopic dermatitis, itching, skin lesions induced by severe itching or atopic dermatitis, systemic sclerosis, pruritic conditions, and pruritis;
diseases of the gastro-intestinal tract and metabolic diseases including diarrhoea, hepatic encephalopathy, hypoglycaemia, gastroesophageal reflux disease (GERD), astrointestinal dysfunction, lower esophageal sphincter (LES) disease, functional gastrointestinal disorders, dyspepsia, vomiting, urinary incontinence, and regurgitation;
diseases of the immune system, including Sjogren's syndrome, systemic lupus erythematosus, and multiple sclerosis (MS);
eye diseases, including eye injuries, eye diseases, eye disorders, glaucoma, retinopathy, and macular degeneration;
diseases of the respiratory tract, including respiratory tract infection, chronic laryngitis, asthma, reflux-related asthma, and lung disease;
migraine; autism; restless leg syndrome (RLS); Tourette syndrome; micturition disorders; neuromuscular disorder in the lower urinary tract; and drug tolerance to opioids.

Further, the invention relates to the use of a compound of Formula **I** as defined above or an optical isomer, polymorph, analog, derivative, prodrug, pharmaceutically-acceptable acid or base addition salt, hydrate, or solvate thereof for the manufacture of a medicament for the prevention and/or treatment of a condition associated with excitotoxicity and malfunctioning of glutamatergic neurotransmission. Such a use includes the use of such a compound for the manufacture of a medicament for the prevention and/or treatment of a condition in an animal including a human being which condition is associated with excitotoxicity and malfunctioning of glutamatergic neurotransmission, including conditions selected from those described earlier in the description.

Moreover, the invention relates to a method for treating or preventing a condition associated with excitotoxicity and malfunctioning of glutamatergic neurotransmission, including conditions selected from those described earlier in the description, such method comprising administering to a living animal, Including a human, a therapeutically effective amount of a compound selected from those of Formula **I** as defined above or an optical isomer, polymorph, analog, derivative, prodrug, pharmaceutically-acceptable acid or base addition salt, hydrate, or solvate thereof.

The compounds of the invention are suitable for administration in monotherapy or for combination therapy with other pharmaceutically active compounds. Examples of suitable other pharmaceutically active compounds include immunomodulators and agents active against central nervous system disorders such as other NMDA agonists or antagonsists including glycine B antagonsists.

A further aspect of the invention relates to such a method wherein the compound is administered in the form of a pharmaceutical composition thereof comprising at least one compound of Formula **I** in combination with one or more pharmaceutically-acceptable diluents, excipients, or carriers.

Further, the invention relates to a pharmaceutical composition comprising as active ingredient at least one compound of Formula **I** as defined above, or an optical isomer, polymorph, analog, derivative, prodrug, pharmaceutically-acceptable acid or base addition salt, hydrate, or solvate thereof, together with one or more pharmaceutically acceptable excipients or vehicles.

The invention also relates to a process for the synthesis or preparation of a compound selected from those of Formula **I** as defined above, comprising reaction of a compound of Formula **II**: 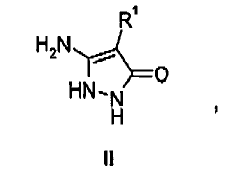 wherein R¹ is as defined for Formula **I** above, with a compound of Formula **III**: 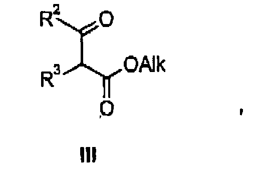 wherein R² and R³ are as defined for Formula **I** above, and Alk represents an alkyl group (e.g., Me, Et), optionally in the presence of base in an appropriate solvent (e.g., sodium hydroxide or potassium hydroxide in aqueous alcohol, sodium ethylate in ethanol, or piperidine in DMF), to yield a compound of Formula **I**, which may be converted, if desired, into an optical isomer, polymorph, analog, derivative, prodrug, pharmaceutically-acceptable salt, hydrate or solvate.

### DETAILED DESCRIPTION OF THE INVENTION

For the purpose of the present invention, the carbon atom content of various hydrocarbon-containing moieties is indicated by a prefix designating the minimum and maximum number of carbon atoms in the moiety, i.e., the prefix Cᵢ₋ⱼ indicates a moiety of the integer "i" to the integer "j" carbon atoms, inclusive. Thus, for example, C₁₋₃alkyl refers to alkyl of one to three carbon atoms, inclusive, (i.e., methyl, ethyl, propyl, and isopropyl), straight and branched forms thereof.

As used herein and as far as it is not defined in a different manner elsewhere in this description or the accompanying claims, the term "C₁₋₆alkyl" represents straight or branched chain alkyl groups having 1, 2, 3, 4, 5 or 6 carbon atoms, examples of such alkyl groups include methyl, ethyl, n-propyl, 2-propyl, n-butyl, 2-butyl, iso-butyl, tert-butyl, n-pentyl, 2-pentyl, 3-pentyl, iso-pentyl, 2-methylbutyl, tert-amyl, n-hexyl, 2-hexyl, 3-hexyl, 1-methylpentyl, 2-methylpentyl, 3-methylpentyl, 4-methylpentyl, 2-dimethylbutyl, 3-dimethylbutyl, 2-ethylbutyl, and 3-ethylbutyl, Further, such alkyl groups may optionally be substituted by one or more fluorine, chlorine and/or bromine atoms and/or one or more carboxy or carbamoyl moieties; examples of halogenated alkyl moieties include -CF₃, -C₂F₅, -CBr₃, and -CCl₃. The term "C₂₋₆alkenyl" represents straight or branched chain alkenyl groups having 2, 3, 4, 5 or 6 carbon atoms. The term "cycloC₃₋₁₂alkyl" represents monocyclic or bicyclic, or tricyclic alkyl groups having 3, 4, 5, 6, 7, 8, 9, 10, 11 or 12 carbon atoms, including cyclopropyl, cyclobutyl, cyclopentyl, cyclohexyl, bicyclo[2.2.1]heptyl and adamantanyl, wherein the "cycloC₃₋₁₂alkyl"-ring is optionally substituted by one or more (e.g., 1, 2, 3, or 4) fluorine, chlorine, and/or bromine atoms. In the context of the present invention the term "di-(C₁₋₆alkyl)amino" refers to an amino moiety in which the nitrogen atom of the amino group is substituted with two C₁-₆alkyl groups, which may be the same or different, as defined above. Examples of di-C₁-₆alkylamino groups include dimethylamino, diethylamino and N-methyl-N-isopropylamino. The term "aryl" represents phenyl or naphthyl, wherein the phenyl or naphthyl group is optionally substituted by one or more (e.g., 1, 2, 3, or 4) substituents, which may be the same or different, selected independently from halogen, amino, hydroxy, nitro, cyano, COOH, trifluoromethyl, C₁-₆alkyl, C₂₋₆alkenyl, C₂₋₆alkynyl, heteroaryl, C₁₋₆alkoxy, difluoromethoxy, trifluoromethoxy, cyclo-C₃₋₁₂alkoxy, aryloxy, heteroaryloxy, aryl-C₁₋₆alkoxy, heteroaryl-C₁₋₆alkoxy, amino-C₁₋₆alkyl, hydroxy-C₁₋₆alkyl, carboxy-C₁₋₆alkyl, carbamoyl-C₁₋₆alkyl, C₁₋₆alkoxy-C₁₋₆alkyl, hydroxy-C₁₋₆alkoxy, C₁₋₆alkoxy-C₁₋₆alkoxy, carboxy-C₁₋₆alkoxy, C₁₋₆alkoxycarbonyl, C₁₋₆alkylamino, cyclo-C₃₋₁₂alkylamino, arylamino, heteroarylamino, aryl-C₁₋₆alkylamino, heteroaryl-C₁₋₆alkylamino, hydroxy-C₁₋₆alkylamino, carboxy-C₁-₆alkylamino, C₁₋₆alkylamino-C₁₋₆alkyl, di-(C₁₋₆alkyl)amino, acylamino, di-(C₁₋₆alkyl)amino-C₁₋₆alkyl, carboxy-C₁₋₆alkylamino-C₁₋₆alkyl, C₁₋₆alkylamino-C₁₋₆alkoxy, di-(C₁₋₆alkyl)amino-C₁₋₆alkoxy, carboxy-C₁₋₆alkylamino-C₁₋₆alkoxy, C₁₋₆alkylsulfonylamino, arylsulfonylamino, C₁₋₆alkylsulfonylamino-C₁₋₆alkyl, C₁₋₆alkyl-aminosulfonyl, di-(C₁₋₆alkyl)aminosulfonyl, arylaminosulfonyl, heteroarylaminosulfonyl, C₁₋₆alkylaminocarbonyl-C₁₋₆alkyl, cyclo-C₃₋₁₂alkylaminocarbonyl-C₁₋₆alkyl, arylaminocarbonyl-C₁₋₆alkyl, heteroarylaminocarbonyl-C₁₋₆alkyl, aryl-C₁₋₆alkylaminocarbonyl-C₁₋₆alkyl, heteroaryl-C₁₋₆alkylaminocarbonyl-C₁₋₆alkyl, hydroxy-C₁₋₆alkylaminocarbonyl-C₁₋₆alkyl, carboxy-C₁₋₆alkylaminocarbonyl-C₁₋₆alkyl, di-(C₁₋₆alkyl)aminocarbonyl-C₁₋₆alkyl, aryl(C₁₋₆alkyl)aminocarbonyl-C₁₋₆alkyl, and heteroaryl(C₁₋₆alkyl)aminocarbonyl-C₁₋₆alkyl. The term "heteroaryl" represents an aromatic 5-6 membered ring comprising one to four heteroatoms selected from oxygen, sulfur and nitrogen or a bicyclic ring system having one 5-6 membered ring comprising one to four heteroatoms selected from oxygen, sulfur and nitrogen fused with a benzene ring or a 5-6 membered ring comprising one to four heteroatoms selected from oxygen, sulfur and nitrogen, wherein the heteroaryl is optionally substituted by one or more (e.g., 1, 2, 3, or 4) substituents, which may be the same or different, selected independently from halogen, amino, hydroxy, nitro, cyano, COOH, trifluoromethyl, C₁₋₆alkyl, C₂₋₆alkenyl, C₂₋₆alkynyl, heteroaryl, C₁₋₆alkoxy, difluoromethoxy, trifluoromethoxy, cyclo-C₃₋₁₂alkoxy, aryloxy, heteroaryloxy, aryl-C₁₋₆alkoxy, hetemaryl-C₁₋₆alkoxy, amino-C₁₋₆alkyl, hydroxy-C₁₋₆alkyl, carboxy-C₁₋₆alkyl, carbamoyl-C₁₋₆alkyl, C₁₋₆alkoxy-C₁₋₆alkyl, hydroxy-C₁₋₆alkoxy, C₁₋₆alkoxy-C₁₋₆alkoxy, carboxy-C₁₋₆alkoxy, C₁₋₆alkoxycarbonyl, C₁₋₆alkylamino, cyclo-C₃₋₁₂alkylamino, arylamino, heteroarylamino, aryl-C₁₋₆alkylamino, heteroaryl-C₁₋₆alkylamino, hydroy-C₁₋₆alkylamino, carboxy-C₁₋₆alkylamino, C₁₋₆alkylamino-C₁₋₆alkyl, di-(C₁₋₆alkyl)amino, acylamino, di-(C₁₋₆alkyl)aminoC₁₋₆alkyl, carboxy-C₁₋₆alkylamino-C₁₋₆alkyl, C₁₋₆alkylamino-C₁₋₆alkoxy, di-(C₁₋₆alkyl)amino-C₁₋₆alkoxy, carboxy-C₁₋₆alkylamino-C₁₋₆alkoxy, C₁₋₆alkylsulfonylamino, arylsulfonylamino, C₁₋₆alkylsulfonylamin-C₁₋₆alkyl, C₁₋₆alkyl-aminosulfonyl, di-(C₁₋₆alkyl)aminosulfonyl, arylaminosulfonyl, heteroarylaminosulfonyl, C₁₋₆alkylaminocarbonyl-C₁₋₆alkyl, cyclo-C₃₋₁₂alkylaminocarbonyl-C₁₋₆alkyl, arylaminocarbonyl-C₁₋₆alkyl, heteroarylaminocarbonyl-C₁₋₆alkyl, aryl-C₁₋₆alkylaminocarbonyl-C₁₋₆alkyl, heteroaryl-C₁₋₆alkylaminocarbonyl-C₁₋₆alkyl, hydroxy-C₁₋₆alkylaminocarbonyl-C₁₋₆alkyl, carboxy-C₁₋₆alkylaminocarbonyl-C₁₋₆alkyl di-(C₁₋₆alkyl)aminocarbonyl-C₁₋₆alkyl, aryl(C₁₋₆alkyl)aminocarbonyl-C₁₋₆alkyl, and heteroaryl(C₁₋₆alkyl)aminocarbonyl-C₁₋₆alkyl; examples of such heteroaryl groups include furyl, thienyl, pyrrolyl, oxazolyl, isoxazolyl, thiazolyl, imidazolyl, pyrazolyl, oxadiazolyl, thiadiazolyl, tetrazolyl, pyridinyl, pyrimidinyl, pyrazinyl, pyridazinyl, benzofuryl, benzothienyl, indolyl, benzimidazolyl, benzoxazolyl, benzothiazolyl, quinolinyl, isoquinolinyl, quinazolinyl, and quinoxalinyl. The term "acyl" represents C₁₋₆alkylcarbonyl, trifluoroacetyl, C₂₋₆alkenylcarbonyl, C₂₋₆alkynylcarbonyl, hydroxy-C₁₋₆alkylcarbonyl, carboxy-C₁₋₆alkylcarbonyl, C₁₋₆alkoxy-C₁₋₆alkylcarbonyl, arylcarbonyl, heteroarylcarbonyl, cyclo-C₃₋₁₂alkylcarbonyl, cyclo-C₃₋₁₂alkyl-C₁₋₆alkylcarbonyl, cyclo-C₃₋₁₂alkoxy-C₁₋₆alkylcarbonyl, aryloxy-C₁₋₆alkylcarbonyl, heteroaryloxy-C₁₋₆alkylcarbonyl, aryl-C₁₋₆alkylcarbonyl, heteroaryl-C₁₋₆alkylcarbonyl, aryl-C₁₋₆alkoxycarbonyl, heteroaryl-C₁₋₆alkoxycarbonyl, arylamino-C₁₋₆alkylcarbonyl; heteroarylamino-C₁₋₆alkylcarbonyl, cyclo-C₃₋₁₂alkylamino-C₁₋₆alkylcarbonyl, carboxy-C₁₋₆alkylamino-C₁₋₆alkylcarbonyl, arylsulfonylamino-C₁₋₆aalkylcarbonyl, C₁₋₆alkylsulfonylamino-C₁₋₆alkylcarbonyl, heterocyclylcarbonyl and heterocyclyl-C₁₋₆alkylearbonyl, The term "heterocyclyl" represents a saturated 4-7 membered heterocycle containing one or two heteroatoms selected from oxygen, sulfur and nitrogen, wherein the heterocyclyl is optionally substituted with one or two oxo moieties, examples of such heterocyclyl groups include azetidinyl, pyrrolidinyl, piperidinyl, azepanyl, tetrahydrofuryl, thiazolidinyl, morpholinyl, thiomorpholinyl, and piperazinyl. The term "halogen" represents fluorine, chlorine, bromine and iodine.

The compounds of the present invention are named according to the IUPAC or CAS nomenclature system. Abbreviations which are well known to one of ordinary skill in the art may be used (e.g. "Ph" for phenyl, "Me" for methyl, "Et" for ethyl, "h" for hour or hours, and "rt" for room temperature).

The term "analog" or "derivative" is used herein in the conventional pharmaceutical sense, to refer to a molecule that structurally resembles a reference molecule (such as 1H,4H-pyrazofo[1,5-a]pyrimidine-2,7-dione), but has been modified in a targeted and controlled manner to replace one or more specific substituents of the reference molecule with an alternate substituent, thereby generating a molecule which is structurally similar to the reference molecule. Synthesis and screening of analogs (e.g., using structural and/or biochemical analysis), to identify slightly modified versions of a known compound which may have improved or biased traits (such as higher potency and/or selectivity at a specific targeted receptor type, fewer side effects, etc.) is a drug design approach that is well known in pharmaceutical chemistry.

In addition, using methods known to those skilled in the art, analogs and derivatives of the compounds of the invention can be created which have improved therapeutic efficacy, *i.e.,* higher potency and/or selectivity at a specific targeted receptor type, either greater or lower ability to penetrate mammalian blood-brain barriers (e.g., either higher or lower blood-brain barrier permeation rate), fewer side effects, etc.

The term "prodrug" is used herein in the conventional pharmaceutical sense, to refer to a molecule which undergoes a transformation *in vivo* (e.g., an enzymatic or chemical transformation) to release an active parent drug. Prodrugs of the compounds of Formula **I** of the present invention may be prepared by chemically modifying a functional group present in the compound of Formula **I** such that the chemically modified compound may undergo a transformation *in vivo* (e.g., enzymatic hydrolysis) to provide the compound of Formula **I**. Examples of functional groups present in the compounds of Formula **I** which may be modified to produce prodrugs include carboxy, hydroxy, amino, and thio groups. Prodrugs of the compounds of Formula **I**. of the present invention may be prepared according to conventional techniques which have been described in the art (see, for example, Stella V., et al., Prodrugs: Challenges and Rewards, AAPS Press/Springer, New York, 2007**).**

The phrase "pharmaceutically acceptable", as used in connection with compositions of the invention, refers to molecular entities and other ingredients of such compositions that are physiologically tolerable and do not typically produce untoward reactions when administered to a mammal (*e.g.,* human). The term "pharmaceutically acceptable" may also mean approved by a regulatory agency of the Federal or a state government or listed in the U.S. Pharmacopeia or other generally recognized pharmacopeia for use in mammals, and more particularly in humans.

Compounds of the present invention may be in the form of pharmaceutically acceptable salts. "Pharmaceutically acceptable salts" refers to those salts which possess the biological effectiveness and properties of the parent compound and which are not biologically or otherwise undesirable. The nature of the salt or isomer is not critical, provided that it is non-toxic and does not substantially interfere with the desired pharmacological activity.

It will be appreciated by those skilled in the art that compounds of the invention having a chiral center may exist in and be isolated in optically active and racemic forms. Some compounds may exhibit polymorphism. It is to be understood that the present invention ecompasses any racemic, optically-active, polymorphic, tautomeric, or stereoisomeric form, or mixture thereof, of a compound of the invention, which possesses the useful properties described herein.

Pure stereoisomeric forms of the compounds and the intermediates of this invention may be obtained by the application of art-known procedures. Diastereomers may be separated by physical separation methods such as selective crystallization and chromatographic techniques, e.g. liquid chromatography using chiral stationary phases. Enantiomers may be separated from each other by selective crystallization of their diastereomeric salts with optically active acids. Alternatively, enantiomers may be separated by chromatographic techniques using chiral stationary phases. Pure stereoisomeric forms may also be derived from the corresponding pure stereoisomeric form of appropriate starting materials, provided that the reaction occurs stereoselectively. Stereoisomeric forms of Formula **I** are obviously intended to be included within the scope of this invention.

As noted above, the present invention encompasses tautomeric forms of compounds of Formula **I**. The compounds of the present invention may, for example, exist in the following tautomeric forms:

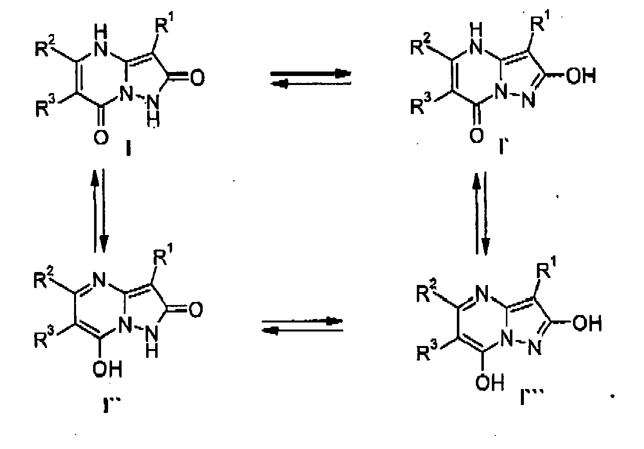

### ADDITION SALTS

For therapeutic use, salts of the compounds of Formula **I** are those wherein the counterion is pharmaceutically acceptable. However, salts of acids and bases, which are non-pharmaceutically acceptable, may also find use, for example, in the preparation and purification of pharmaceutically acceptable compounds. All salts whether pharmaceutically acceptable or not are included within the ambit of the present invention. The pharmaceutically acceptable salts as mentioned above are meant to comprise the therapeutically active non-toxic salt forms, which the compounds of Formula **I** are able to form. The latter can conveniently be obtained by treating the base form with such appropriate acids as inorganic acids, e.g. hydrohalic acids such as hydrochloric, hydrobromic and the like; sulfuric acid; nitric acid; phosphoric acid and the like; or organic acids such as acetic, propanoic, hydroxyacetic, 2-hydroxypropanoic, oxopropanoic, oxalic, malonic, succinic, maleic, fumaric, malic, tartaric, 2-hydroxy-1,2,3-propanetricarboxylic, methanesulfonic, ethanesulfonic, benzenesulfonic, 4-methylbenzenesulfonic, cyclohexanesulfonic, 2-hydroxybenzoic, 4-amino-2-hydroxybenzoic and like acids. Conversely, the salt may be converted to the free base by treatment with alkali.

### PHARMACEUTICAL COMPOSITIONS

The active ingredients of the compounds of the present invention, together with one or more conventional adjuvants, carriers, or diluents, may be placed into the form of pharmaceutical compositions and unit dosages thereof, and in such form may be employed as solids, such as coated or uncoated tablets or filled capsules, liquids, such as solutions, suspensions, emulsions, elixirs, or capsules filled with the same, or thin films/flash doses, all for oral use; in the form of suppositories or capsules for rectal administration or in the form of sterile injectable solutions for parenteral (including intravenous or subcutaneous) use. Such pharmaceutical compositions and unit dosage forms thereof may comprise conventional or new ingredients in conventional or special proportions, with or without additional active compounds or principles, and such unit dosage forms may contain any suitable effective amount of the active ingredient of the compounds of the present invention commensurate with the intended daily dosage range to be employed. Tablets containing one (1) to one hundred (100) milligrams of active ingredient or, more broadly, zero point five (0.5) to five hundred (500) milligrams per tablet, are accordingly suitable representative unit dosage forms.

The term "excipient" applied to pharmaceutical compositions of the invention refers to an adjuvant, carrier, diluent, or vehicle with which a compound of the present invention is administered. Such pharmaceutical excipients may be sterile or non-sterile excipients commonly used for the formulation and production of solid, semi solid, liquid and sterile pharmaceutical compositions. These excipients may also be liquids, such as water, saline solutions, aqueous dextrose solutions, aqueous glycerol solutions, and oils, including those of petroleum, animal, vegetable or synthetic origin, such as peanut oil, soybean oil, mineral oil, sesame oil and the like. A.R. Gennaro, 20th Edition, describes suitable pharmaceutical carriers in "Remington: The Science and Practice of Pharmacy". The excipients may also be combinations of solids and liquids.

### METHOD OF TREATING

Due to their high degree of activity and their low toxicity, together presenting a most favorable therapeutic index, the active principles of the invention may be administered to a subject, e.g., a living animal (including a human) body, in need thereof, for the treatment, alleviation, or amelioration, palliation, or elimination of an indication or condition which is susceptible thereto, or representatively of an indication or condition set forth elsewhere in this application, including concurrently, simultaneously, or together with one or more pharmaceutically-acceptable excipients, carriers, or diluents, including in the form of a pharmaceutical composition thereof, whether by oral, rectal, or parental (including intravenous and subcutaneous) or in some cases even topical route, in an effective amount. Suitable dosage ranges are 1-1000 milligrams daily, optionally 10-500 milligrams daily, and optionally 50-500 milligrams daily, depending as usual upon the exact mode of administration, form in which administered, the indication toward which the administration is directed, the subject involved and the body weight of the subject involved, and the preference and experience of the physician or veterinarian in charge.

The term "treat" is used herein to mean to relieve or alleviate at least one symptom of a disease in a subject. Within the meaning of the present invention, the term "treat" also denotes to arrest, delay the onset (*i.e.*, the period prior to clinical manifestation of a disease) and/or reduce the risk of developing or worsening a disease.

The term "therapeutically effective" applied to dose or amount refers to that quantity of a compound or pharmaceutical composition that is sufficient to result in a desired activity upon administration to a living animal body in need thereof.

The compounds of the present invention may be administered orally, topically, parenterally, or mucosally (*e.g.*, buccally, by inhalation, or rectally) in dosage unit formulations containing conventional non-toxic pharmaceutically acceptable carriers. It is usually desirable to use the oral route. The compounds of the present invention may be administered orally in the form of a capsule, a tablet, or the like (see Remington: The Science and Practice of Pharmacy, 20th Edition). The orally administered medicaments may be administered in the form of a time-controlled release vehicle, including diffusion-controlled systems, osmotic devices, dissolution-controlled matrices, and erodible/degradable matrices.

For oral administration in the form of a tablet or capsule, the glycine B antagonist active component may be combined with a non-toxic, pharmaceutically acceptable excipients such as binding agents (*e.g.*, pregelatinized maize starch, polyvinylpyrrolidone or hydroxypropyl methylcellulose); fillers (*e.g.*, lactose, sucrose, glucose, mannitol, sorbitol and other reducing and non-reducing sugars, microcrystalline cellulose, calcium sulfate, or calcium hydrogen phosphate); lubricants (*e*.*g*., magnesium stearate, talc, or silica, steric acid, sodium stearyl fumarate, glyceryl behenate, calcium stearate, and the like); disintegrants (*e.g.*, potato starch or sodium starch glycolate); or wetting agents (*e.g.*, sodium lauryl sulphate), coloring and flavoring agents, gelatin, sweeteners, natural and synthetic gums (such as acacia, tragacanth or alginates), buffer salts, carboxymethylcellulose, polyethyleneglycol, waxes, and the like. For oral administration in liquid form, the glycine B antagonist active components may be combined with non-toxic, pharmaceutically acceptable inert carriers (*e.g.,* ethanol, glycerol, water), suspending agents (*e.g.*, sorbitol syrup, cellulose derivatives or hydrogenated edible fats), emulsifying agents (*e.g*., lecithin or acacia), non-aqueous vehicles (*e.g*., almond oil, oily esters, ethyl alcohol or fractionated vegetable oils), preservatives (*e.g.*, methyl or propyl-p-hydroxybenzoates or sorbic acid), and the like. Stabilizing agents such as antioxidants (BHA, BHT, propyl gallate, sodium ascorbate, citric acid) may also be added to stabilize the dosage forms.

The tablets may be coated by methods well known in the art. The compounds of the present invention may be also introduced in beads, microspheres or microcapsules, *e.g*., fabricated from polyglycolic acid/lactic acid (PGLA). Liquid preparations for oral administration may take the form of, for example, solutions, syrups, emulsions or suspensions, or they may be presented as a dry product for reconstitution with water or other suitable vehicle before use. Preparations for oral administration may be suitably formulated to give controlled or postponed release of the active compound.

The compounds of the present invention may also be administered in the form of liposome delivery systems, such as small unilamellar vesicles, large unilamellar vesicles and multilamellar vesicles. Liposomes can be formed from a variety of phospholipids, such as cholesterol, stearylamine or phosphatidylcholines, as is well known.

The compounds of the present invention may also be delivered by the use of monoclonal antibodies as individual carriers to which the compound molecules are coupled. The instant compounds may also be coupled with soluble polymers as targetable drug carriers. Such polymers include polyvinyl-pyrrolidone, pyran copolymer, polyhydroxy-propyl methacrylamide-phenol, polyhydroxy-ethyl-aspartamide-phenol, or polyethyleneoxide-polylysine substituted with palmitoyl residues. Furthermore, the instant compounds may be coupled to a class of biodegradable polymers useful in achieving controlled release of a drug, for example, polylactic acid, polyglycolic acid, copolymers of polylactic and polyglycolic acid, polyepsilon caprolactone, polyhydroxybutyric acid, polyorthoesters, polyacetals, polyhydropyrans, polycyanoacrylates, and cross-linked or amphipathic block copolymers of hydrogels.

For administration by inhalation, the compounds of the present invention may be conveniently delivered in the form of an aerosol spray presentation from pressurized packs or a nebulizer, with the use of a suitable propellant, *e.g.,* dichlorodifluoromethane, trichlorofluoromethane, dichlorotetrafluoroethane, carbon dioxide, or other suitable gas. In the case of a pressurized aerosol, the dosage unit may be determined by providing a valve to deliver a metered amount. Capsules and cartridges of, *e.g.,* gelatin for use in an inhaler or insufflator can be formulated containing a powder mix of the compound and a suitable powder base such as lactose or starch.

The formulations comprising the compounds of the present invention may be delivered parenterally, *i.e.,* by intravenous (i.v.), intracerebroventricular (i,c.v.), subcutaneous (s.c.), intraperitoneal (i.p.), intramuscular (i.m.), subdermal (s.d.), or intradermal (i.d.) administration, by direct injection, via, for example, bolus injection or continuous infusion. Formulations for injection can be presented in unit dosage form, *e.g.,* in ampoules or in multi-dose containers, with an added preservative. The compositions can take such forms as excipients, suspensions, solutions, or emulsions in oily or aqueous vehicles, and can contain formulatory agents such as suspending, stabilizing and/or dispersing agents. Alternatively, the active ingredient of the compounds of the present invention can be in powder form for reconstitution with a suitable vehicle, *e.g.,* sterile pyrogen-free water, before use.

The compounds of the present invention may also be formulated for rectal administration, *e.g.,* as suppositories or retention enemas (*e.g.*, containing conventional suppository bases such as cocoa butter or other glycerides).

The compositions comprising glycine B antagonists of the present invention may, if desired, be presented in a pack or dispenser device, which may contain one or more unit dosage forms containing the active ingredient and/or may contain different dosage levels to facilitate dosage titration. The pack may, for example, comprise metal or plastic foil, such as a blister pack. The pack or dispenser device may be accompanied by instructions for administration. The glycine B antagonists of the present invention formulated in a compatible pharmaceutical carrier may also be prepared, placed in an appropriate container, and labeled for treatment of an indicated condition.

As disclosed herein, the dose of the components in the compositions of the present invention is determined to ensure that the dose administered continuously or intermittently will not exceed an amount determined after consideration of the results in test animals and the individual conditions of a patient. A specific dose naturally varies depending on the dosage procedure, the conditions of a patient or a subject animal such as age, body weight, sex, sensitivity, feed, dosage period, drugs used in combination, seriousness of the disease. The appropriate dose and dosage times under certain conditions can be determined by the test based on the above-described indices but may be refined and ultimately decided according to the judgment of the practitioner and each patient's circumstances (age, general condition, severity of symptoms, sex, etc.) according to standard clinical techniques.

Toxicity and therapeutic efficacy of the compositions of the invention can be determined by standard pharmaceutical procedures in experimental animals, *e.g.,* by determining the LD₅₀ (the dose lethal to 50% of the population) and the ED₅₀ (the dose therapeutically effective in 50% of the population). The dose ratio between therapeutic and toxic effects is the therapeutic index and it may be expressed as the ratio LD₅₀/ED₅₀. Compositions that exhibit large therapeutic indices are preferred.

The following schemes describe the preparation of the compounds of the present invention. **Scheme 1** describes the preparation of compounds of Formula **I,** and **Scheme 2** describes additional methods for preparing starting materials and intermediates for use in the preparation of compounds of Formula I. All of the starting materials are prepared by procedures described in these schemes, by procedures well known to one of ordinary skill in organic chemistry or can be obtained commercially. All of the final compounds of the present invention are prepared by procedures described in these charts or by procedures analogous thereto, which procedures would be well known to one of ordinary skill in organic chemistry. All of the variables used in the schemes are as defined below or as in the claims. 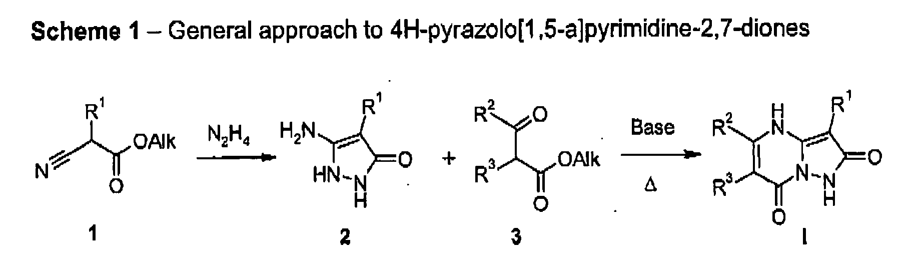 Alk = alkyl (e.g., Me, Et)

The compounds of general Formula I may be prepared by reacting a compound of the formula **2** with the compound of the formula **3** in the presence of an appropriate base as, for example, sodium hydroxide or potassium hydroxide in aqueous alcohol solution, or sodium ethylate in ethyl alcohol, or piperidine in dimethylformamide. Alternatively, reaction may be performed by heating the reagents **2** and **3** without solvent. Aminopyrazole **2** may be prepared by reaction of alkyl cyanoacetate **1** with hydrazine or with hydrazine hydrate, or by cyclization of intermediate hydrazide of substituted cyanoacetic acid, respectively. Alternative methods for preparing aminopyrazole derivatives **(2)** are shown in **Scheme 2.**

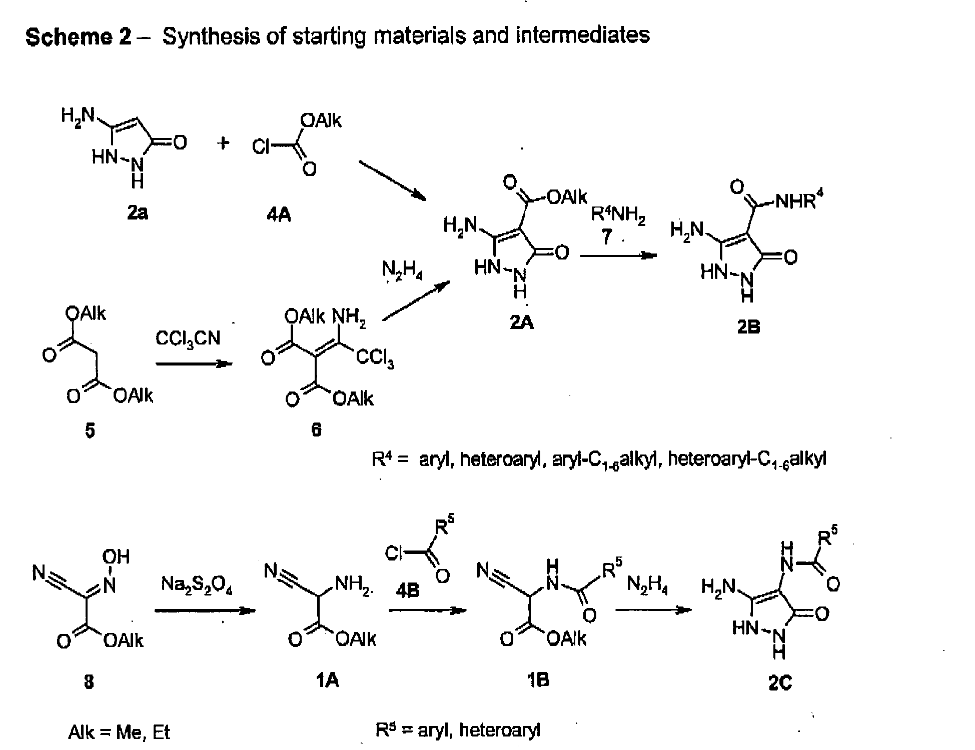

4-(Substituted)carbamoyl derivatives of aminopyrazole **2 (2B)** may be prepared by amidation of alkyl ester of aminopyrazolonecarboxylic acid **2A** with amine 7. Compounds of the formula **2A** may be prepared according to well known procedures, such as by acylation of 5-amino-2,3-dihydro-1H-pyrazol-3-one **(2a)** with alkyl chloroformate **4a** [Papini et al. Gazzetta Chimica Italiana. 1954, 84, 769-780.] or by reacting malonate derivative **6** with hydrazine [Gavrilenko B. B.; Miller S. I. J. Org. Chem. 1975, 40(19), 2720-2724.]. 4-(Substituted)amido derivatives of aminopyrazole **2 (2C)** may be prepared according to procedures shown in **Scheme 2.** Reduction of alkyl cyanoacetate derivative **8** with sodium ditionite provides alkyl aminocyanoacetate **1A** which may be acylated by a compound **4B** to yield an alkyl acylamino-cyanoacetate 1B [Caille J. C., Didierlaurent S., Lefrancois D., Lelièvre M. H., Sury C., Aszodi J.. Synthesis. 1995, 6, 635-637. and Cabon G., Gaucher B., Gegout A., Heulle S., Masquelin T. Chimia. 2003, 57(5), 248- 254.]. Compounds of formula **1B** may be treated with hydrazine to yield aminopyrazolone derivatives **2C.**

It will be appreciated that in the above transformations it may be necessary or desirable to protect any sensitive groups in the molecule of the compound in question in order to avoid undesirable side reactions. The reaction products may be isolated and purified by standard laboratory techniques, such as extraction, chromatography and crystallization. Products isolated as a free base may be further converted into a hydrochloride or any other pharmaceutically acceptable salt according to known procedures. Products isolated as a free carboxylic acid may be converted into sodium salt or any other pharmaceutically acceptable salt according to known procedures.

It will be apparent to those skilled in the art that the described synthetic procedures are merely representative in nature and that alternative synthetic processes are known to one of ordinary skill in organic chemistry.

### EXPERIMENTAL PART

The compounds and their preparation of the present invention will be better understood in connection with the following examples, which are intended as an illustration of and not a limitation upon the scope of the invention.

Hereinafter, "DMF" is defined as N,N-dimethylformamide, "HCl" as hydrochloric acid, "DMSO" as dimethyl sulfoxide, "NH₄OH" as ammonium hydroxide solution, "MeCN" as acetonitrile, "AcOH" as acetic acid, "EtOH" as ethanol, "MeOH" as methanol and SiO₂ as silica gel.

### Example 1

### 5,6,7,8-Tetrahydro-1H,4H-pyrazolo[5,1-b]quinazoline-2,9-dione

5-Amino-2,3-dihydro-1H-pyrazol-3-one is dissolved in aqueous EtOH containing KOH. To this solution is added ethyl 2-oxocyclohexane-1-carboxylate and the mixture is stirred under reflux for 1 h 30 min. After cooling to rt, water is added and the mixture is acidified by addition of acetic acid to pH∼5. The resulting precipitate is collected on a filter, washed with water and diethyl ether, and dried to give the title compound in moderate yield. Mp >250 °C (decomp.); ¹H-NMR (200 MHz, DMSO-d₆), δ (ppm): 1.67 (m, 4H): 2.34 (m, 2H); 2.52 (m, 2H); 5.22 (s, 1H) and 11.62 (br s, 1H). LC MS m/z 206 (M+1).

### Example 2

### 3-Phenyl-5,6,7,8-tetrahydro-1H,4H-pyrazolo[5,1-b]quinazoline-2,9-dlone

In analogy to the procedure described in **Example 1,** 5-amino-4-phenyl-2,3-dihydro-1H-pyrazol-3-one is reacted with ethyl 2-oxocyclohexane-1-carboxylate to give the title compound in moderate yield. ¹H-NMR (400 MHz, DMSO-d₆), δ (ppm): 1.68 (m, 4H): 2.39 (t, 5.6 Hz, 2H); 2.58 (m, 2H); 7.09 (br s, 1H); 10.89 (br s, 1H) and 11.27 (br s, 1H). LC MS m/z 282 (M+1).

### Example 3

### 2,9-Dioxo-1,2,4,5,6,7,8,9-octahydro-pyrazolo[5,1-b]quinazoline-3-carboxylic acid phenylamide

A mixture of ethyl 2,9-dioxo-1,2,4,5,6,7,8,9-octahydropyrazolo[5,1-b]quinazoline-3-carboxylate (100 mg, 0.36 mmol) and aniline (66 µl, 0.72 mmol, 2.0 equiv.) in DMF (3 ml) is irradiated at 150°C (intensity normal, cooling on) for 1 hour. The mixture is poured into brine and the resulting solid is filtered off. The residue is washed with aqueous acetic acid, water and ethanol followed by EtOAc and ether, and dried to give the title compound (70.9 mg, 60%) as a tan colored solid. mp: 328-330°C (decomp.); LC-MS m/z 323 (M-H-); ¹H-NMR (400 MHz, DMSO-d₆), δ (ppm): 1.65-1.71 (m, 4H): 2.38 (t, 5.4 Hz, 2H); 2.70 (t, 5.5 Hz, 2H); 7.04 (t, 8 Hz, 1H); 7.32 (t, 8 Hz, 2H); 7.62 (d, 8Hz, 2H); 10.14 (s, 1H) and 12.13 (brs, 1H).

### Example 4

### N-(2,9-Dioxo-1,2,4,5,6,7,8,9-octahydro-pyrazolo[5,1-b]quinazolin-3-yl)-benzamide

A mixture of N-(5-amino-3-oxo-2,3-dihydro-1H-pyrazol-4-yl)-benzamide and ethyl 2-oxocyclohexane-1-carboxylate is stirred for 4.5 h (TLC control) at 140-150 °C. The mixture is then cooled to rt and stirred for 10 min with Et₂O and filtered. The resulting solid is washed with Et₂O and MeOH and dried to give the title compound in 73% yield. ¹H-NMR (400 MHz, DMSO-d₆), δ (ppm): 1.64-1.68 (m, 4H): 2.36 (m, 2H); 2.52 (m, 2H); 7.46-7.55 (m, 3H); 7.99 (d, 7.2 Hz, 2H); 9.44 (s, 1H); 10.95 (br s, 1H) and 11.32 (s, 1H). LC MS m/z 325 (M+1).

### Example 5

### Ethyl 2,9-dioxo-1,2,4,5,6,7,8,9-octahydro-pyrazolo[5,1-b]quinazoline-3-carboxylate

In analogy to the procedure described in Example 4, ethyl 5-amino-3-oxo-2,3-dihydro-1H-pyrazole-4-carboxylate is reacted with ethyl 2-oxocyclohexane-1-carboxylate at 150-160 °C to give after chromatographical separation the title compound in moderate yield. ¹H-NMR (400 MHz, DMSO-d₆), δ (ppm): 1.24 (t, 7 Hz, 3H); 1.65 (m, 4H), 2.35 (t, 6 Hz, 2H); 2.67 (t, 6 Hz, 2H); 4.21 (q, 7 Hz, 2H) and 11.11 (s, 1H). LC MS m/z 278 (M+1).

### Example 6

### 2,9-Dioxo-1,2,4,5,6,7,8,9-octahydro-pyrazolo[5,1-b]quinazoline-3-carboxylic acid (4-chloro-phenyl)-amlde

In analogy to the procedure described in **Example 3,** ethyl 2,9-dioxo-1,2,4,5,6,7,8,9-octahydro-pyrazolo[5,1-b]quinazoline-3-carboxylate is reacted with 4-chloro-phenylamine o give the title compound in good yield. ¹H-NMR (400 MHz, DMSO-d6): δ (ppm) 1.65-1.71 (m, 4H), 2.38 (t, 2H), 2.67 (t, 2H), 7.36 (d, 2H), 7.66 (d, 2H), 10.43 (br s, 1H), 11.99 (br s, 1H).

### Example 7

### 2,9-Dioxo-1,2,4,5,6,7,8,9-octahydro-pyrazolo[5,1-b]quinazoline-3-carboxylic acid (3-chloro-phenyl)-amide

In analogy to the procedure described in **Example 3,** ethyl 2,9-dioxo-1,2,4,5,6,7,8,9-octahydro-pyrazolo[5,1-b]quinazoline-3-carboxylate is reacted with 3-chloro-phenylamine to give the title compound in good yield. ¹H-NMR (400 MHz, DMSO-d6): δ (ppm) 1.65-1.71 (m, 4H), 2.38 (t, 2H), 2.56 (s, 1H), 2.67 (t, 2H), 7.04-7.07 (m, 1H), 7.30-7.39 (m, 2H), 8.02 (s, 1H), 10.64 (br s, 1H), 11.84 (br s, 1H).

### Example 8

### 2,9-Dioxo-1,2,4,5,6,7,8,9-octahydro-pyrazolo[5,1-b]quinazoline-3-carboxylic acid (2-chloro-phenyl)-amide

In analogy to the procedure described in **Example 3,** ethyl 2,9-dioxo-1,2,4,5,6,7,8,9-octahydro-pyrazolo[5,1-b]quinazoline-3-carboxylate is reacted with 2-chloro-phenylamine to give the title compound in good yield.

### Example 9

### 2,9-Dioxo-1,2,4,5,6,7,8,9-octahydro-pyrazolo[5,1-b]quinazoline-3-carboxylic acid benzylamide

In analogy to the procedure described in **Example 3,** ethyl 2,9-dioxo-1,2,4,5,6,7,8,9-octahydro-pyrazolo[5,1-b]quinazoline-3-carboxylate is reacted with benzylamine to give the title compound in good yield. ¹H-NMR (400 MHz, DMSO-d6), δ (ppm) 1.62-1.71 (m, 4H), 2.34-2.39 (m, 2H), 2.67-2.71 (m, 2H), 4.48 (d, 2H), 7.22-7.26 (m, 1H), 7.30-7.34 (m, 4H), 8.06 (br s, 1H), 11.31 (br s, 1H), 12.51 (br s, 1H).

### Example 10

### 3-((E)-Styryl)-5,6,7,8-tetrahydro-1H,4H-pyrazolo[5,1-b]quinazoline-2,9-dione

A solution of equimolar amounts of 5,6,7,8-tetrahydro-1H,4H-pyrazolo[5,1-b]quinazoline-2,9-dione and phenylacetaldehyde in acetonitrile is stirred at 65 °C for 72 h in the presence of piperidine and acetic acid to give after chromatographical separation the title compound in moderate yield.

### Example 11

### 3-[(E)-2-(4-Chloro-phenyl)-vinyl]-5,6,7,8-tetrahydro-1H,4H-pyrazolo[5,1-b]quinazoline-2,9-dione

In analogy to the procedure described in **Example 10,** 5,6,7,8-tetrahydro-1H,4H-pyrazolo[5,1-b]quinazoline-2,9-dione is reacted with (4-chloro-phenyl)-acetaldehyde to give the title compound in good yield.

### Example 12

### 3-[(E)-2-(3-Chloro-phenyl)-vinyl]-5,6,7,8-tetrahydro-1H,4H-pyrazolo[5,1-b]quinazoline-2,9-dione

In analogy to the procedure described in **Example 10,** 5,6,7,8-tetrahydro-1 H,4H-pyrazolo[5,1-b]quinazoline-2,9-dione is reacted with (3-chloro-phenyl)-acetaldehyde to give the title compound in good yield.

### Example 13

### 3-[(2,9-Dioxo-1,2,4,5,6,7,8,9-octahydro-pyrazolo[5,1-b]quinazoline-3-carbonyl)-amino]-benzoic acid

In analogy to the procedure described in **Example 3,** ethyl 2,9-dioxo-1,2,4,5,6,7,8,9-octahydro-pyrazolo[5,1-b]quinazoline-3-carboxylate is reacted with 3-amino-benzoic acid to give the title compound in good yield.

### Example 14

### Benzyl 2,9-dioxo-1,2,4,5,6,7,8,9- octahydro-pyrazolo[5,1-b]quinazoline-3-carboxylate

In analogy to the procedure described in **Example 4,** benzyl 5-amino-3-oxo-2,3-dihydro-1H-pyrazole-4-carboxylate is reacted with ethyl 2-oxocyclohexane-1-carboxylate under solvent free conditions to give the title compound in moderate yield.

### Example 15

### 2,9-Dioxo-1,2,4,5,6,7,8,9-octahydro-pyrazolo[5,1-b]quinazoline-3-carboxylic acid (4-mothoxy-phenyl)-amide

In analogy to the procedure described in **Example 3,** ethyl 2,9-dioxo-1,2,4,5,6,7,8,9-octahydro-pyrazolo[5,1-b]quinazoline-3-carboxylate is reacted with 4-methoxy-phenylamine to give the title compound in good yield.

### Example 16

### 2,9-Dioxo-1,2,4,5,6,7,8,9-octahydro-pyrazolo[5,1-b]quinazoline-3-carboxylic acid pyridin-3-ylamide

In analogy to the procedure described in **Example 3,** ethyl 2,9-dioxo-1,2,4,5,6,7,8,9-octahydro-pyrazolo[5,1-b]quinazoline-3-carboxylate is reacted with pyridin-3-ylamine to give the title compound in good yield.

### Example 17

### N-(2,9-Dioxo-1,2,4,9-tetrahydro-pyrazolo[5,1-b]quinazolin-3-yl)-benzamide

### a) 3-Amino-1H,4H-pyrazolo[5,1-b]qutnazoline-2,9-dione

A solution of NaNO₂ (2.2 mmol) in water (2.0 ml) was added to a suspension of 1H,4H-pyrazolo[5,1-b]quinazoline-2,9-dione (2 mmol) in acetic acid (5 ml). The mixture was stirred at room temperature for 1.5 hours, then sodium dithionite (85 wt%, 6 mmol) was added and the mixture was stirred for another hour. The mixture was concentrated *in vacuo* and the residue was triturated with water. The resulting suspension was filtered off and the residue was washed with water, followed by a minor amount of ethanol and ether to give the title compound in good yield.

### b) N-(2,9-Dioxo-1,2,4,9-tetrahydro-pyrazolo[5,1-b]quinazolin-3-yl)-benzamide

To a solution of 3-amino-1H,4H-pyrazolo[5,1-b]quinazofine-2,9-dione in 5% aqueous NaOH solution was added benzoyl chloride (at least double excess) and the resulting mixture was stirred at room temperature for 1 h. The mixture was acidified with aqueous 2N HCl solution and concentrated to dryness. The residue was triturated with water and the suspension was filtered off. The residue was washed with water and ethanol followed by heptane and ether to give the title product as an off white solid.

### Example 18

### N-(2,9-Dioxo-1,2,4,9-tetrahydro-pyrazolo[5,1-b]quinazolin-3-yl)-4-methoxy-benzamide

In analogy to the procedure described in **Example 17b,** 3-amino-1H,4H-pyrazolo[5,1-b]quinazoline-2,9-dione is reacted with 4-methoxy-benzoyl chloride to give the title compound in moderate yield.

### Example 19

### N-(2,9-Dioxo-1,2,4,9-tetrahydro-pyrazolo[5,1-b]quinazolin-3-yl)-2-fiuoro-benzamide

In analogy to the procedure described in **Example 17b,** 3-amino-1H,4H-pyrazolo[5,1-b]quinazoline-2,9-dione is reacted with 2-f!uoro-benzoyl chloride to give the title compound in moderate yield.

### Example 20

### N-(2,9-Dioxo-1,2,4,9-tetrahydro-pyrazolo[5,1-b]quinazolin-3-yl)-3-chloro-benzamide

In analogy to the procedure described in **Example 17b,** 3-amino-1H,4H-pyrazolo[5,1-b]quinazoline-2,9-dione is reacted with 3-chloro-benzoyl chloride to give the title compound in moderate yield.

### Example 21

### 2,9-Dioxo-1,2,4,5,6,7,8,9-octahydro-pyrazolo[5,1-b]quinazoline-3-carboxylic acid pyridin-2-ylamide

In analogy to the procedure described in **Examples 3,** ethyl 2,9-dioxo-1,2,4,5,6,7,8,9-octahydro-pyrazolo[5,1-b]quinazoline-3-carboxylate is reacted with pyridin-2-ylamine to give the title compound in good yield.

### Example 22

### 2,9-Dioxo-1,2,4,5,6,7,8,9-octahydro-pyrazolo[5,1-b]quinazoline-3-carboxylicacid pyridin-4-ylamide

In analogy to the procedure described in **Example 3,** ethyl 2,9-dioxo-1,2,4,5,6,7,8,9-octahydro-pyrazolo[5,1-b]quinazoline-3-carboxylate is reacted with pyridin-4-ylamine to give the title compound in moderate yield.

### Example 23

### 2,9-Dioxo-1,2,4,5,6,7,8,9-octahydro-pyrazolo[5,1-b]quinazoline-3-carboxylic acid furan-3-ylamide

In analogy to the procedure described in **Example 3,** ethyl 2,9-dioxo-1,2,4,5,6,7,8,9-octahydro-pyrazolo[5,1-b]quinazoline-3-carboxylate is reacted with furan-3-ylamine to give the title compound in moderate yield.

### Example 24

### 2,9-Dioxo-1,2,4,5,6,7,8,9-octahydro-pyrazolo[5,1-b]quinazoline-3-carboxylic acid (1H-pyrrol-2-yl)-amide

In analogy to the procedure described in **Example 3,** ethyl 2,9-dioxo-1,2,4,5,6,7,8,9-octahydro-pyrazoio[5,1-b]quinazoline-3-carboxylate is reacted with 1H-pyrrol-2-ylamine to give the title compound in moderate yield.

### Example 25

### 2,9-Dioxo-1,2,4,5,6,7,8,9-octahydro-pyrazolo[5,1-b]qulnazoline-3-carboxylic acid (3-hydroxy-phenyl)-amide

In analogy to the procedure described in **Example 3,** ethyl 2,9-dioxo-1,2,4,5,6,7,8,9-octahydro-pyrazolo[5,1-b]quinazoline-3-carboxylate is reacted with 3-aminophenol to give the title compound in moderate yield.

### Example 26

### 2,9-Dioxo.1,2,4,5,6,7,8,9-octahydro-pyrazolo[5,1-b]quinazoline-3-carboxylic acid (4-hydroxy-phenyl)-amide

In analogy to the procedure described in **Example 3,** ethyl 2,9-dioxo-1,2,4,5,6,7,8,9-octahydro-pyrazolo[5,1-b]quinazoline-3-carboxylate is reacted with 4-aminophenol to give the title compound in moderate yield.

### Example 27

### 2,9-Dioxo-1,2,4,5,6,7,8,9-octahydro-pyrazolo[5,1-b]quinazoline-3-carboxylic acid (2-fluoro-phenyl)-amide

In analogy to the procedure described in **Example 3,** ethyl 2,9-dioxo-1,2,4,5,6,7,8,9-octahydro-pyrazolo[5,1-b]quinazoline-3-carboxylate is reacted with 2-fluoro-phenylamine to give the title compound in moderate yield.

### Example 28

### 2,9-Dioxo-1,2,4,5,6,7,8,9-octahydro-pyrazolo[5,1-b]quinazoline-3-carboxylic acid (2-hydroxy-phenyl)-amide

In analogy to the procedure described in **Example 3,** ethyl 2,9-dioxo-1,2,4,5,6,7,8,9-octahydro-pyrazolo[5,1-b]quinazoline-3-carboxylate is reacted with 2-amino-phenol to give the title compound in moderate yield.

### Example 29

### N-(2,9-Dioxo-1,2,4,5,6,7,8,9-octahydro-pyrazolo[5,1-b]quinazolin-3-yl)-2-fluoro-benzamide

In analogy to the procedure described in **Example 4,** N-(5-amino-3-oxo-2,3-dihydro-1H-pyrazo)-4-yl)-2-fluoro-benzamide is reacted with ethyl 2-oxocyclohexane-1-carboxylate to give the title compound in moderate yield.

### Example 30

### N-(2,9-Dioxo-1,2,4,5,6,7,8,9-octahydro-pyrazolo[5,1-b]quinazolin-3-yl)-2-hydroxy-benzamide

In analogy to the procedure described in **Example 4,** N-(5-amino-3-oxo-2,3-dihydro-1H-pyrazol-4-yl)-2-hydroxy-benzamide is reacted with ethyl 2-oxocyclohexane-1-carboxylate to give the title compound in moderate yield.

### Example 31

### 3-[(E)-2-(2-Fluoro-phenyl)-vinyl]-5,6,7,8-tetrahydro-1H,4H-pyrazolo[5,1-b]quinazoline-2,9-dione

In analogy to the procedure described in **Example 10,** 5,6,7,8-tetrahydro-1H,4H-pyrazolo[5,1-b]quinazoline-2,9-dione is reacted with (2-fluoro-phenyl)-acetaldehyde to give the title compound in good yield.

### Example 32

### 2,9-Dioxo-1,2,4,9-tetrahydro-pyrazolo[5,1-b]quinazoline-3-carboxylic acid

Ethyl 2,9-dioxo-1,2,4,9-tetrahydro-pyrazolo[5,1-b]quinazoline-3-carboxylate is hydrolyzed by an excess of lithium hydroxide hydrate to give after acidification with aqueous hydrochloric acid the title compound in moderate yield.

### Example 33

### 2,9-Dioxo-1,2,4,9-tetrahydro-pyrazolo[5,1-b]quinazoline-3-carboxylic acid phenylamide

In analogy to the procedure described in **Example 3,** ethyl 2,9-dioxo-1,2,4,9-tetrahydro-pyrazolo[5,1-b]quinazoline-3-carboxylate is reacted with aniline to give the title compound in moderate yield.

### Example 34

### 2,9-Dioxo-1,2,4,9-tetrahydro-pyrazolo[5,1-b]quinazoline-3-carboxylic acid (4-methoxy-phenyl)-amide

In analogy to the procedure described in **Example 3,** ethyl 2,9-dioxo-1,2,4,9-tetrahydro-pyrazolo[5,1-b]quinazoline-3-carboxylate is reacted with 4-methoxy-phenylamine to give the title compound in moderate yield.

### Example 35

### 2,9-Dioxo-9,2,4,9-tetrahydro-pyrazolo[5,1-b]quinazoline-3-carboxylic acid (2-fluoro-phonyl)-amide

In analogy to the procedure described in **Example 3,** ethyl 2,9-dioxo-1,2,4,9-tetrahydro-pyrazolo[5,1-b]quinazoline-3-carboxylate is reacted with 2-fluoro-phenylamine to give the title compound in moderate yield.

### Example 36

### 2,9-Dioxo-1,2,4,9-tetrahydro-pyrazolo[5,1-b]quinazoline-3-carboxylic acid (3-chloro-phenyl)-amide

In analogy to the procedure described in **Example 3,** ethyl 2,9-dioxo-1,2,4,9-tetrahydro-pyrazolo[5,1-b]quinazoline-3-carboxylate is reacted with 3-chloro-phenylamine to give the title compound in moderate yield.

### Example 37

### 3-(4-Fluoro-phenylamino)-5,6,7,8- tetrahydro-1H,4H-pyrazolo[5,1-b]quinazoline-2,9-dione

In analogy to the procedure described in **Example 4,** 5-amino-4-(4-fluoro-phenylamino)-2,3-dihydro-1H-pyrazol-3-one is reacted with ethyl 2-oxocyclohexane-1-carboxylate to give the title compound in moderate yield.

### Example 38

### 3-(4-Fluoro-phenylamino)-1H,4H-pyrazolo[5,1-b]quinazoline-2,9-dione

2-Amino-benzoic acid hydrazide is reacted with equimolar amount of diethyl 2-(4-fluoro-phenylamino)-malonate in conditions analogous to described in Example 3 to give the title compound in moderate yield.

### Example 39

### N-(2,9-Dioxo-1,2,4,5,6,7,8,9-octahydro-pyrazolo[5,1-b]quinazolin-3-yl)-4-methyl-benzenesulfonamide

In analogy to the procedure described in **Example 4,** N-(5-amino-3-oxo-2,3-dihydro-1H-pyrazol-4-yl)-4-methyl-benzenesulfonamide is reacted with ethyl 2-oxocyclohexane-1-carboxylate to give the title compound in moderate yield.

### Example 40

### N-(2,9-Dioxo-1,2,4,9-tetrahydro-pyrazolo[5,1-b]quinazolin-3-yl)-4-methyl-benzenesulfonamide

In analogy to the procedure described in **Example 17b,** 3-amino-1H,4H-pyrazolo[5,1-b]quinazoline-2,9-dione is reacted with 4-methyl-benzenesulfonyl chloride to give the title compound in moderate yield.

### Example 41

### 2-(2,9-Dioxo-1,2,4,5,6,7,8,9-octahydro-pyrazolo[5,1-b]quinazolin-3-ylamino)-3-phenyl-propionic acid

3-Amino-5,6,7,8-tetrahydro-1H,4H-pyrazolo[5,1-b]quinazoline-2,9-dione (prepared from 5,6,7,8-tetrahydro-1H,4H-pyrazolo[5,1-b]quinazoline-2,9-dione by analogy to the procedure described in **Example 17a**) is reacted with ethyl 2-oxo-3-phenyl-propionate in the presence of sodium cyanoborohydride to give the title compound after hydrolysis of ethyl ester group.

### Example 42

### 2-(2,9-Dioxo-1,2,4,9-tetrahydro-pyrazolo[5,1-b]quinazolin-3-ylamino)-3-phenyl-propionic acid

In analogy to the procedure described in **Example 41,** 3-amino-1H,4H-pyrazolo[5,1-b]quinazoline-2,9-dione is reacted with ethyl 2-oxo-3-phenyl-propionate the presence of cyanoborohydride to give the title compound after hydrolysis of ethyl ester group.

**Table 1**

| | | |
|---|---|---|
| 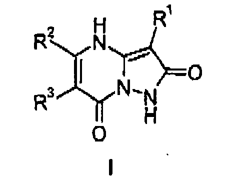 | | |

| **Example No.** | **R¹** | **R² + R³** |
|---|---|---|
| **1** | H | (CH₂)₄ |
| **2** | Ph | (CH₂)₄ |
| **3** | CONHPh | (CH₂)₄ |
| **4** | NHCOPh | (CH₂)₄ |
| **5** | CO₂Et | (CH₂)₄ |
| **6** | 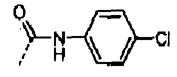 | (CH₂)₄ |
| **7** | 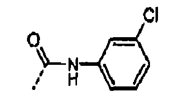 | (CH₂)₄ |
| **8** | 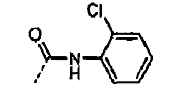 | (CH₂)₄ |
| **9** | CONHCH₂Ph | (CH₂)₄ |
| **10** | CH=CHPh | (CH₂)₄ |
| **11** | 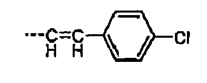 | (CH₂)₄ |
| **12** | 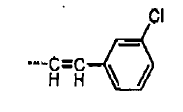 | (CH₂)₄ |
| **13** | 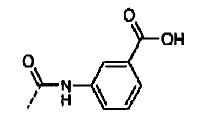 | (CH₂)₄ |
| **14** | COOCH₂Ph | (CH₂)₄ |
| **15** | 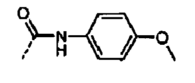 | (CH₂)₄ |
| **16** | 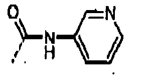 | (CH₂)₄ |
| **17** | NHCOPh | -CH=CH-CH=CH- |
| **18** | 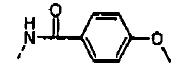 | -CH=CH-CH=CH- |
| **19** | 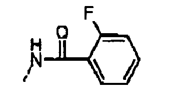 | -CH=CH-CH=CH- |
| **20** | 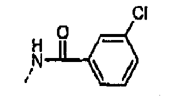 | -CH=CH-CH=CH- |
| **21** | 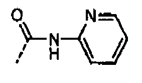 | (CH₂)₄ |
| **22** | 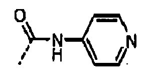 | (CH₂)₄ |
| **23** | 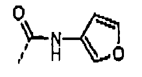 | (CH₂)₄ |
| **24** | 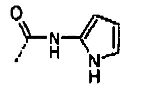 | (CH₂)₄ |
| **25** | 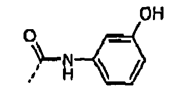 | (CH₂)₄ |
| **26** | 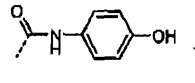 | (CH₂)₄ |
| **27** | 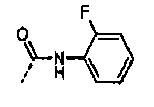 | (CH₂)₄ |
| **28** | 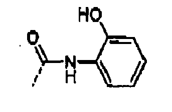 | (CH₂)₄ |
| **29** | 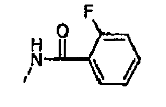 | (CH₂)₄ |
| **30** | 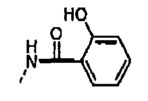 | (CH₂)₄ |
| **31** | 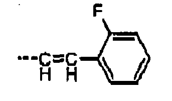 | (CH₂)₄ |
| **32** | COOH | -CH=CH-CH=CH- |
| **33** | CONHPh | -CH=CH-CH=CH- |
| **34** | 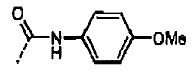 | -CH=CH-CH=CH- |
| **35** | 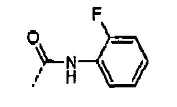 | -CH=CH-CH=CH- |
| **36** | 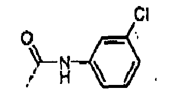 | -CH=CH-CH=CH- |
| **37** | 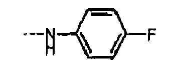 | (CH₂)₄ |
| **38** | 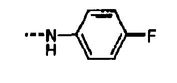 | -CH=CH-CH=CH- |
| **39** | 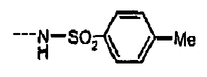 | (CH₂)₄ |
| **40** | 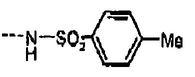 | -CH=CH-CH=CH- |
| **41** | 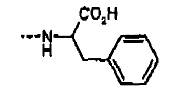 | (CH₂)₄ |
| **42** | 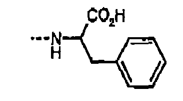 | -CH=CH-CH=CH- |

### EXAMPLES OF REPRESENTATIVE PHARMACEUTICAL COMPOSITIONS

With the aid of commonly used solvents, auxiliary agents and carriers, the reaction products can be processed into tablets, coated tablets, capsules, drip solutions, suppositories, injection and infusion preparations, and the like and can be therapeutically applied by the oral, rectal, parenteral, and additional routes. Representative pharmaceutical compositions follow.
(a) Tablets suitable for oral administration, which contain the active ingredient, may be prepared by conventional tabletting techniques:
(b) For suppositories, any usual suppository base may be employed for incorporation thereinto by usual procedure of the active ingredient, such as a polyethyleneglycol which is a solid at normal room temperature but which melts at or about body temperature.
(c) For parental (including intravenous and subcutaneous) sterile solutions, the active ingredient together with conventional ingredients in usual amounts are employed, such as for example sodium chloride and double-distilled water q.s., according to conventional procedure, such as filtration, aseptic filling into ampoules or IV-drip bottles, and autoclaving for sterility.

Other suitable pharmaceutical compositions will be immediately apparent to one skilled in the art.

### FORMULATION EXAMPLES

The following examples are again given by way of illustration only and are not to be construed as limiting.

### EXAMPLE 1

### Tablet Formulation

A suitable formulation for a tablet containing 10 milligrams of active ingredient is as follows:

| | mg |
|---|---|
| Active Ingredient | 10 |
| Lactose | 61 |
| Microcrystalline Cellulose | 25 |
| Talcum | 2 |
| Magnesium stearate | 1 |
| Colloidal silicon dioxide | 1 |

### EXAMPLE 2

### Tablet Formulation

Another suitable formulation for a tablet containing 100 mg is as follows:

| | mg |
|---|---|
| Active Ingredient | 100 |
| Polyvinylpyrrolidone, crosslinked | 10 |
| Potato starch | 20 |
| Polyvinylpyrrolidone | 19 |
| Magnesium stearate | 1 |
| Microcrystalline Cellulose | 50 |
| Film coated and colored. | |

The film coating material consists of:

| | |
|---|---|
| Hypromellose | 10 |
| Microcryst. Cellulose | 5 |
| Talcum | 5 |
| Polyethylene glycol | 2 |
| Color pigments | 5 |

### EXAMPLE 3

### Capsule Formulation

A suitable formulation for a capsule containing 50 milligrams of active ingredient is as follows:

| | mg |
|---|---|
| Active Ingredient | 50 |
| Com starch | 26 |
| Dibasic calcium phosphate | 50 |
| Talcum | 2 |
| Colloidal silicon dioxide | 2 |

filled in a gelatin capsule.

### EXAMPLE 4

### Solution for injection

A suitable formulation for an injectable solution is as follows:

| | | |
|---|---|---|
| Active ingredient | mg | 10 |
| Sodium chloride | mg | q.s. |
| Water for Injection | mL | add 1.0 |

### EXAMPLE 5

### Liquid oral formulation

A suitable formulation for 1 liter of an oral solution containing 2 milligrams of active ingredient in one milliliter of the mixture is as follows:

| | mg |
|---|---|
| Active Ingredient | 2 |
| Saccharose | 250 |
| Glucose | 300 |
| Sorbitol | 150 |
| Orange flavor | 10 |
| Colorant | q.s. |
| Purified water | add 1000 mL |

### EXAMPLE 6

### Liquid oral formulation

Another suitable formulation for 1 liter of a liquid mixture containing 20 milligrams of active ingredient in one milliliter of the mixture is as follows:

| | G |
|---|---|
| Active Ingredient | 20.00 |
| Tragacanth | 7.00 |
| Glycerol | 50.00 |
| Saccharose | 400.00 |
| Methylparaben | 0.50 |
| Propylparaben | 0.05 |
| Black currant-flavor | 10.00 |
| Soluble Red color | 0.02 |
| Purified water | add 1000 mL |

### EXAMPLE 7

### Liquid oral formulation

Another suitable formulation for 1 liter of a liquid mixture containing 2 milligrams of active ingredient in one milliliter of the mixture is as follows:

| | G |
|---|---|
| Active Ingredient | 2 |
| Saccharose | 400 |
| Bitter orange peel tincture | 20 |
| Sweet orange peel tincture | 15 |
| Purified water | add 1000 mL |

### EXAMPLE 8

### Aerosol formulation

180 g aerosol solution contain:

| | G |
|---|---|
| Active Ingredient | 10 |
| Oleic acid | 5 |
| Ethanol | 81 |
| Purified Water | 9 |
| Tetrafluoroethane | 75 |

15 ml of the solution are filled into aluminum aerosol cans, capped with a dosing valve, purged with 3.0 bar.

### EXAMPLE 9

### TDS formulation

100 g solution contain:

| | G |
|---|---|
| Active Ingredient | 10.0 |
| Ethanol | 57.5 |
| Propyleneglycol | 7.5 |
| Dimethylsulfoxide | 5.0 |
| Hydroxyethylcellulose | 0.4 |
| Purified water | 19.6 |

1.8 ml of the solution are placed on a fleece covered by an adhesive backing foil. The system is closed by a protective liner which will be removed before use.

### EXAMPLE 10

### Nanoparticle formulation

10 g of polybutylcyanoacrylate nanoparticles contain:

| | G |
|---|---|
| Active Ingredient | 1.00 |
| Poloxamer | 0.10 |
| Butylcyanoacrylate | 8.75 |
| Mannitol | 0.10 |
| Sodium chloride | 0.05 |

Polybutylcyanoacrylate nanoparticles are prepared by emulsion polymerization in a water/0.1 N HCl/ethanol mixture as polymerizsation medium. The nanoparticles in the suspension are finally lyophilized under vacuum.

### PHARMACOLOGY

The active principles of the present invention, and pharmaceutical compositions thereof and method of treating therewith, are characterized by unique and advantageous properties, rendering the "subject matter as a whole", as claimed herein, unobvious. The compounds and pharmaceutical compositions thereof exhibit, in standard accepted reliable test procedures, the following valuable properties and characteristics:

### METHODS

### BINDING ASSAYS FOR THE CHARACTERIZATION OF GLYCINE B ANTAGONIST PROPERTIES

### [³H]MDL-105,519 Displacement Studies

For the evaluation of the binding affinity of the test compounds on the glycine binding pocket of the NMDA receptor, [³H]-MDL-105,59 (GE Healthcare, Freiburg, Germany) displacement studies are performed using a 96-well plate robotic platform. MDL-105,519 (Baron et al., J Pharmacol Exp Ther 1996, 279(1), 62-68; Baron et al., European Journal of Pharmacology, 1997, 323(2-3), 181-192; Hoffner & Wanner, Neuroscience Letters, 1997, 226(2), 79-82) is a selective, high affinity antagonist at the NMDA receptor glycine site.

### Preparation of cortical membranes:

Tissue preparation is performed according to Foster & Wong (Br J Pharmacol, 1987, 91, 403-409) with some modifications. Anaesthetised male Sprague-Dawley rats (200-250 g, Janvier, Le Genest-Isle, France) are decapitated and their brains removed rapidly. The cortex is dissected out and processed as described by Parsons, et al. (J Pharmacol Exp Ther, 1997, 283(3), 1264-1275). For isolation of the cell membranes, the cortices are homogenized in 20 volumes of ice-cold 0.32 M sucrose (Sigma-Aldrich, Taufkirchen, Germany) using a glass-Teflon homogenizer. The homogenate is centrifuged at 1000 x g for 10 minutes, the pellet is discarded and the supernatant centrifuged at 20,000 x g for 20 minutes. The resulting pellet is re-suspended in 20 volumes of distilled water and centrifuged for 20 minutes at 8000 x g. The supernatant and the buffy coat are then centrifuged three times (48,000 x g for 20 minutes) in the presence of 50 mM Tris-HCl, pH 8.0 (assay buffer). All centrifugation steps are carried out at 4°C. After resuspension in 5 volumes of 50 mM Tris-HCl, pH 7.5, the membrane suspension is frozen rapidly at -80°C. On the day of assay, the membranes are thawed and washed four times by resuspension in 50 mM Tris-HCl, pH 7.5, and centrifugation at 48,000 x g for 20 minutes. The final pellet is suspended in assay buffer. The amount of protein in the final membrane preparation is determined according to the method of Lowry, et al. (J. Biological Chemistry, 1951, 193, 256-275) with some modifications (Hartree, Analytical Biochemistry, 1972, 48, 422-427). The final protein concentration used for our studies is 400 µg/ ml.

### Displacement studies

A robotic system designed for binding assays (Tecan Deutschland GmbH, Crailsheim, Germany) is loaded with the membrane solution, solutions for bound control (buffer/DMSO 20%), unlabeled glycine (1 mM) for evaluation of non-specific binding, all compounds to be tested (at 20-fold concentrations), radioligand and respective 96-well plates.

Before performing displacement studies, saturation experiments are performed to determine the equilibrium dissociation constant (K_{d}) of [³H]-MDL-105,519, which is a parameter for the affinity of the radioligand to the binding site. The protein/receptor concentration is held constant whereas the amount of specific bound radioligand is determined using increasing concentrations of ligand.

On the basis of the saturation experiments, a final [³H]-MDL-105,519 concentration of 2 nM is selected. Firstly, the assay plates are loaded with membrane solution and are shaken at 4°C. The mother plates are then prepared by pipetting the compounds into assay buffer/20% DMSO to obtain the desired final concentrations (dose response curve with five different concentrations, e.g. 10, 3, 1, 0.3, and 0.1 µM). After transferring radioligand into the assay plates, the compounds are added (including the bound and the non-specific binding control). The final DMSO concentration is 1 %. The assay plates are incubated and shaken at 4°C for 1 h, before the mixture is exhausted as rapidly as possible via a vacuum manifold using the Multiscreen HTS glass fibre (type B) filter plates (Millipore, Schwalbach, Germany) under a constant vacuum of 450 mbar. The membranes are washed four times with cold assay buffer (100 µL). 50 µL of Ultima Gold scintillation cocktail (PerkinElmer, Rodgau-Jügesheim, Germany) is added to the wet filter plates and incubated at room temperature overnight before counting the disintegration per minutes using a liquid scintillation counter (MicroBeta, PerkinElmer, Rodgau-Jügesheim, Germany).

### Analysis of data

For the evaluation of the binding affinity of the test compound to the glycine B binding site and its potency to displace [³H]-MDL-105,519, the measured radioactivity of the radioligand alone is set as 100 % bound control and the non-specific binding of the radioligand (which could not displaced by glycine, 1 mM) represented the 0 % control. The residual radioactivity after displacement of the test compound (n = 5) is then corrected with respect to the set controls.

### FUNCTIONAL SCREENING FOR THE CHARACTERIZATION OF GLYCINE B ANTAGONIST PROPERTIES

Antagonistic potencies of the test compounds are functionally evaluated using electrophysiological whole cell patch-clamp recordings and/or fluorometric intracellular Ca²⁺-imaging (FLIPR) screens.

### Whole cell patch-clamp recordings

### Preparation and cultivation of rat hippocampal neurons

Cell preparation is performed as described by Parsons, et al, (Neuropharmacology, 1998, 37(6), 719-727). The female Sprague-Dawiey rat is anaesthetised by placing it in a saturated CO₂-euthanasia chamber under further quiet CO₂-influx. Under these conditions the rat loses consciousness after a few seconds and is then sacrificed by cervical dislocation. After opening the abdominal cavity, embryos (E20) are removed and stored in ice cold Ca²⁺- and Mg²⁺-free Hank's Buffered Salt Solution (pH 7.3), containing 4 g/l glucose (HBSS-CMF). Hippocampi are then isolated from the brains of at least 8 embryos after decapitation, transferred into ice cold HBSS-CMF and washed 3 to 4 times.

Hippocampi are pre-incubated for 8 min with a 0.66% trypsin (Sigma-Aldrich) and 0.1% (20 U/ml) DNAase solution (Sigma-Aldrich) in Ca²⁺-free Phosphate Buffered Saline (PBS-CF) and washed 3 times with HBSS-CMF. Cells are then mechanically dissociated by trituration in a PBS-CF solution containing 0.05% (10 U/ml) DNAase and 0.3% of the trypsin inhibitor ovomucoid (all from Sigma-Aldrich). The cells are then centrifuged at 180 x g for 10 minutes, and the cell pellet re-suspended in basal Minimum Essential Medium (MEM, Invitrogen, Karlsruhe, Germany), again carefully triturated to ensure maximal dissociation and finally plated in the flexiPERM inserts (Thermo Fisher Scientific, Langenselbold, Germany) at a density of 15 x 10³ cells/cm² (0.5 ml/insert) onto poly-DL-omithine (Sigma) and mouse laminin (Invitrogen) pre-coated plastic petri dishes. After 1 hour the cells become attached to the bottom of the dish and the inserts may be removed. The cells are then nourished with 2 ml MEM supplemented with 5% foetal calf serum (FCS) and 5% horse serum and incubated at 37°C with 95% air and 5% CO₂ at 95% humidity. After 4 days *in vitro* (DIV) further glial mitosis is inhibited by adding 10 µl of AraC (5 µM endconcentration). The medium is completely exchanged after an additional 2 DIV and again, but only partly (50 %), after 8 DIV. The cells are used for electrophysiological recordings after 11-15 DIV.

### Evaluation of peripheral antagonistic potencies

For the peripheral glycine B site antagonistic potency evaluation, compounds are functionally tested using dorsal root ganglia (DRG) neurons, modified from Li et al. (Pain, 2004, 109, 443-452).

### Whole cell patch clamp recordings

Cells are visualised using an inverted microscope and selected for patching based upon their position and morphology. Voltage clamp recordings are made in the whole cell configuration of the patch clamp technique at a holding potential of -70 mV with the aid of an EPC-10 amplifier in combination with pipette manipulator. Patch clamp pipettes are pulled from borosilicate glass using a horizontal puller (P-97 Puller, Sutter Instruments, USA) and, when filled with intracellular solution, have resistances of 1 - 4 MΩ.

Solutions are delivered via a home-made gravity driven very fast perfusion system (< 10 ms) including valves to switch flow on and off in combination with a stepper motor-driven double-barrelled theta glass application pipette in order to expose cells to either agonist-free or agonist-containing solutions in presence or absence of antagonist.

The intracellular solution used consists of: 120 mM CsCl, 10 mM EGTA, 1 mM MgCl₂, 200 µM CaCl₂, 10 mM glucose and 22 mM tetraethyl ammonium chloride (TEA-CL). The corresponding extracellular bath solution contains: 140 mM NaCl, 3 mM KCl, 10 mM glucose, 10 mM HEPES, 1.5 mM CaCl₂ and 4.5 mM sucrose (all from Sigma-Aldrich) pH 7.3, and is supplemented with 0.3 µM tetrodotoxin (TTX, Tocris, Bristol, U.K.) to block voltage-activated sodium channels and 0.25 µM bicuculline (Sigma-Aldrich) to block GABA_{A} receptors.

For the determination of concentration-dependency of blockade, 5 control traces are recorded with application of NMDA (200 µM) and D-Serine (1 µM) for 5 seconds in order to reduce the effect of rundown, then the highest concentration of the test-substance is applied for 1 minute before applying the agonists for 5 seconds in the presence of antagonist. Three recordings are made in the presence of the antagonist and 3 recovery traces are recorded after it's removal. The procedure is repeated for three to four further concentrations of antagonist with declining concentrations e.g. 10, 3, 1, 0.3, and 0.1 µM. For the final recovery, agonists are again applied five times after wash-out of the test substance.

### Analysis of data

Data are analysed using TIDA 5.0 (Heka, Lambrecht, Germany). With the help of Microsoft Excel, data are pooled and finally GraFit software (Erithacus Software Ltd., Surrey, U.K.) is used to fit the data e.g. with the four parameter logistic equation for determining IC₅₀ values. For all data points, the value given is the mean ± S.E.M. (standard error of the mean) of results from at least 4 individual cells per concentration.

### Calcium FLIPR studies

### Preparation and cultivation of rat cortical neurons

Primary neurons are prepared from cortices of embryonal rats at day 17 of pregnancy as described by Dichter (Brain Res., 1987, 149, 279). Sprague-Dawley rat embryos (E 17) are decapitated and neocortices are dissected, trypsinized and carefully triturated. The cell suspension is plated on poly-D-lysine pre-coated 96-well Plates (Greiner, Frickenhausen, Germany) at a cell density of 55.000 cells /well. The neurons are cultivated in Neurobasal media containing B27-Supplement and 0.5 µM L-Glutamine (Biochrom) at 37°C in a humidified atmosphere of 5% CO₂ / 95% air. Medium is exchanged completely at day 4 and to 50% on day 7. At the time of experiments neurons are 11-13 days in vitro.

### Calcium FLIPR studies

The increase of intracellular calcium after stimulation with 30 µM NMDA and 1 µM D-Serine is measured using the fluorometric imaging plate reader (FLIPR) and the Calcium-4-Kit (both Molecular Devices, Ismaning, Germany). Prior to addition of agonist or antagonist (5 different concentrations, n = 5) the medium is aspirated and cells are washed once before loading with 150 µL of loading buffer (1 h at room temperature), consisting of Ca-4 sensitive dye reconstituted in extracellular bath solution, pH 7.3. Subsequently, plates are transferred to FLIPR to detect increases in intracellular calcium after the addition of agonist, measured as relative fluorescence units (RFU). Antagonists are pre-incubated with the cells for 10 min at room temperature before the addition of the agonist and co-agonist.

### Data analysis

The fluorescence signal increase after addition of agonist reflects the increase of intracellular calcium. Inconsistencies in the amount of cells per well are normalised by using the spatial uniformity correction of the FLIPR software (Screenworks, Molecular Devices). The mean of replicated temporal data (n=5) is calculated and used for graphical representation. For the evaluation of the antagonistic potency, the calcium changes in response to different concentrations of antagonist are determined using an area under the curve (AUC) calculation. All responses (RFU-values) are determined as percentage of control (= maximum response at 30 µM NMDA and 1 µM D-Serine). IC₅₀ values are calculated according the four parameter logistic equation using GraFit (Erithacus Software).

Compounds of the present invention have an IC₅₀ range of about 0.5 nM to about 100 µM.

### CONCLUSIONS

In conclusion, from the foregoing, it is apparent that the present invention provides novel, valuable, and unpredictable applications and uses of the compounds of the present invention, which compounds comprise the active principle according to the present invention, as well as novel pharmaceutical compositions thereof and methods of preparation thereof and of treating therewith, all possessed of the foregoing more specifically-enumerated characteristics and advantages.

The high order of activity of the active agents of the present invention and compositions thereof, as evidenced by the tests reported, is indicative of utility based on their valuable activity in lower animals. Clinical evaluation in human beings has not been completed, however. It will be clearly understood that the distribution and marketing of any compound or composition falling within the scope of the present invention for use in human beings will of course have to be predicated upon prior approval by governmental agencies, such as the U.S. Federal Food and Drug Administration, which are responsible for and authorized to pass judgment on such questions.

The instant pyrazolopyrimidine derivatives represent a novel class of glycine B antagonists. In view of their potency, they will be useful therapeutics in a wide range of disorders, including CNS disorders, which involve excessive glutamate induced excitation.

These compounds accordingly find application in the treatment of the following disorders of a living animal body, especially a human: pain, including acute pain, chronic pain, allodynia, hyperalgesia, visceral pain, phantom pain, post-operative pain, neuropathic pain, peripheral neuropathy including, for example peripheral neuropathy induced by nociception, inflammation, ischemia, viral infection (HZV), traumatic and other mechanical nerve injury, cancer, diabetes mellitus, HIV infection, fibromyalgia, trigeminus neuralgia, inflammatory bowel diseases (IBD), irritative bowel syndrome (IBS), arthritis including rheumatoid arthritis, osteoarthritis (degenerative joint disease), multiple sclerosis (MS) and gout (metabolic arthritis)

These compounds also find application in the treatment of the following disorders of a living animal body, especially a human: acute insults, including cerebral ischemia, cerebral infarct, brain oedema, anoxia, inner ear insult, inner ear insult in tinnitus, head or brain or spinal cord trauma, head or brain or spinal cord injuries, trauma, sound- or drug-induced inner ear insult, ischaemia resulting from cardiac arrest or stroke or bypass operations or transplants, acute pain, hypoxia, perinatal hypoxia, and ischaemia;
chronic insults, such as neurodegenerative disorders, including Morbus Huntington, Alzheimer's disease Creutzfeld-Jakob's syndrome/disease, bovine spongiform encephalopathy (BSE) prion related infections, diseases involving mitochondrial dysfunction, diseases involving β-amyloid and/or tauopathy, Down's syndrome, motor neuron diseases, amyotrophic lateral sclerosis (ALS), olivoponto-cerebellar atrophy, Parkinson's disease, Neuronal Ceroid Lipofuscinosis, AIDS dementia complex, AIDS-related dementia, dementia related to HIV infections, HIV-1 encephalopathy, AIDS encephalopathy, Korsakoff syndrome, vascular dementia, and corticobasal degeneration;
neurological disorders, including tinnitus, hearing loss, sound- or drug-induced tinnitus, haloperidol-induced dyskinesias, dopaminomimetic-induced dyskinesias, chorea, Huntington's chorea, athetosis, dystonia, stereotypy, ballism, tardive dyskinesias, tic disorder, spasmodic torticollis, blepharospasm, focal and generalized dystonia, nystagmus, Parkinson's dementia, mild cognitive impairment, cognitive deficits in various forms of mild cognitive impairment, cognitive deficits in various forms of dementia, dementia pugilistica, vascular and frontal lobe dementia, cognitive impairment, learning impairment, L-dopa-induced dykinesias, L-dopa-induced dykinesias in Parkinson's disease therapy, dyskinesias, dyskinesia in Huntington's disease, drug induced dyskinesias, neuroleptic-induced dyskinesias, neurodegenerative cerebellar ataxias, centrally induced neuropathic pain, convulsions, epileptic convulsions, epilepsy, temporal lobe epilepsy, myoclonic epilepsy, tremor, dementia in Alzheimer's disease, dementia in Korsakoff syndrome, dementia, hereditary cerebellar ataxias, sleep disorders, movement disorders, essential tremor, muscle spasms, and spasticity;
psychological/psychiatric disorders, including generalized anxiety disorder, obsessive-compulsive disorder, panic disorder, posttraumatic stress disorder, social phobia,
phobic disorders, substance-induced anxiety disorder, delusional disorder, schizoaffective disorder, schizophreniform disorder, substance-induced psychotic disorder, delirium, post-operative cognitive deficit (POCD), cognitive impairment, learning impairment, anxiety disorders, panic disorders, anxiety and panic disorders, social anxiety disorder (SAD), attention deficit hyperactivity disorder (ADHD), attention deficit syndrome (ADS), dementia, posttraumatic stress disorder (PTSD), schizophrenia, positive or cognitive or negative symptoms of schizophrenia, major depressive disorder, major depression, depression, bipolar manic-depressive disorder, sleep disorders, agoraphobia, bulimia nervosa, eating disorders, obesity, obesity-related disorders, obesity abuse, food addiction, binge eating disorders, and hyperactivity in children;
drug/alcohol abuse, including craving (e.g., for drugs of abuse), abuse, addiction, nicotine addiction, nicotine abuse, alcohol addiction, alcohol abuse, opiate addiction, opiate abuse, cocaine addiction, cocaine abuse, amphetamine addiction, and amphetamine abuse;
skin diseases, including atopic dermatitis, itching, skin lesions induced by severe itching or atopic dermatitis, systemic sclerosis, pruritic conditions, and pruritis;
diseases of the gastro-intestinal tract and metabolic diseases, including diarrhoea, hepatic encephalopathy, hypoglycaemia, gastroesophageal reflux disease (GERD), gastrointestinal dysfunction, lower esophageal sphincter (LES) disease, functional gastrointestinal disorders, dyspepsia, vomiting, urinary incontinence, and regurgitation;
diseases of the immune system, including Sjogren's syndrome, systemic lupus erythematosus, and multiple sclerosis (MS);
eye diseases, including eye injuries, eye diseases, eye disorders, glaucoma, retinopathy, and macular degeneration;
diseases of the respiratory tract, including respiratory tract infection, chronic laryngitis, asthma, reflux-related asthma, and lung disease;
migraine; autism; restless leg syndrome (RLS); Tourette syndrome; micturition disorders; neuromuscular disorder in the lower urinary tract; and drug tolerance to opioids.

The method-of-treating a living animal body with a compound of the invention, for the inhibition of progression or alleviation of the selected ailment therein, is as previously stated by any normally- accepted pharmaceutical route, employing the selected dosage which is effective in the alleviation of the particular ailment desired to be alleviated.

Use of the compounds of the present invention in the treatment of a living animal for inhibition of progression or alleviation of selected ailments or conditions, particularly ailments or conditions susceptible to treatment with a glycine B is carried out in the usual manner comprising the step of admixing an effective amount of a compound of the invention with a pharmaceutically-acceptable diluent, excipient, or carrier, and the method-of-treating, pharmaceutical compositions, and use of a compound of the present invention in the manufacture of a medicament.

Representative pharmaceutical compositions prepared by admixing the active ingredient with a suitable pharmaceutically-acceptable excipient, diluent, or carrier, include tablets, capsules, solutions for injection, liquid oral formulations, aerosol formulations, TDS formulations, and nanoparticle formulations, thus to produce medicaments for oral, injectable, or dermal use, also in accord with the foregoing.

The present invention is not to be limited in scope by the specific embodiments described herein. Indeed, various modifications of the invention in addition to those described herein will become apparent to those skilled in the art from the foregoing description.

All patents, applications, publications, test methods, literature, and other materials cited herein are hereby incorporated by reference.

## Claims

1. A compound selected from those of Formula I: 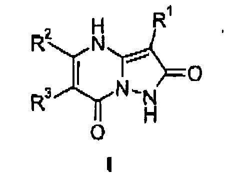 wherein
R¹ represents hydrogen, trifluoromethyl, C₁₋₆alkyl, COOH, C₁₋₆alkoxycarbonyl, hydroxy C₁₋₆alkoxycarbonyl, aryl-C₁₋₆alkoxycarbonyl, heteroaryl-C₁₋₆alkoxycarbonyl, aryl, heteroaryl, aryl-C₁₋₆alkyl, heteroaryl-C₁₋₆alkyl, cyclo-C₅₋₁₂alkyl-C₁₋₆alkyl, aryl-C₂₋₆alkenyl, heteroaryl-C₂₋₆alkenyl, arylamino, heteroarylamino, aryl-C₁₋₆alkylamino; heteroaryl-C₁₋₆alkylamino; acylamino, arylsulfonylamino, C₁₋₆alkylaminocarbonyl, cyclo-C₃₋₁₂alkylaminocarbonyl, arylaminocarbonyl, heteroarylaminocarbonyl, aryl-C₁₋₆alkylaminocarbonyl, heteroaryl-C₁₋₆alkylaminocarbonyl, hydroxy-C₁₋₆alkylaminocarbonyl, di-(C₁₋₆alkyl)aminocarbonyl, aryl(C₁₋₆alkyl)aminocarbonyl, heteroaryl(C₁₋₆alkyl)aminocarbonyl, hydmxy-C₁₋₆alkyl, C₁-₆alkoxy-C₁₋₆alkyl, carboxy-C₁₋₆alkyl, carbamoyl-C₁₋₆alkyl, amino-C₁₋₆alkyl, acylamino-C₁₋₆alkyl, arylamino-C₁₋₆alkyl, aryloxy-C₁₋₆alkyl, C₁₋₆alkylsulfonylamino-C₁₋₆alkyl, arylsulfonylamino-C₁₋₆alkyl, C₁₋₆alkylaminacarbonyl-C₁₋₆alkyl, cyclo-C₃₋₁₂alkylaminocarbonyl-C₁₋₆alkyl, arylaminocarbonyl-C₁₋₆alkyl, heteroarylaminocarbonyl-C₁₋₆alkyl, aryl-C₁₋₆alkylaminocarbonyl-C₁₋₆alkyl, heteroaryl-C₁₋₆alkylaminocarbonyl-C₁₋₆alkyl, hydroxy-C₁₋₆alkylaminocarbonyl-C₁-₆alkyl, carboxy-C₁₋₆alkylaminocarbonyl-C₁₋₆alkyl, di-(C₁₋₆alkyl)aminocarbonyl-C₁₋₆alkyl, aryl(C₁₋₆alkyl)aminocarbonyl-C₁₋₆alkyl, heteroaryl(C₁₋₆alkyl)aminocarbonyl-C₁₋₆alkyl, aryl-heterocyclyl-C₁₋₆alkyl, or heteroaryl-heterocyclyl-C₁₋₆alkyl; and
R² and R³ together represent -(CH₂)ₙ- wherein n is 3, 4, or 5;
or R² and R³ together with the C=C bond to which they are attached represent a phenyl ring or a 5-, 6- or 7-membered ring which may be unsaturated, or partly unsaturated, and wherein the ring in addition to nitrogen atom may contain heteroatom selected from sulfur, oxygen and nitrogen and may be substituted by one or more substituents selected from C₁₋₆alkyl and halogen;
wherein
the term "aryl" represents phenyl or naphthyl, or phenyl substituted by one or more substituents selected independently from halogen, amino, hydroxy, nitro, cyano, COOH, trifluoromethyl, C₁₋₆alkyl, C₂₋₆alkenyl, C₂₋₆alkynyl, heteroaryl, C₁₋₆alkoxy, difluoromethoxy, trifluoromethoxy, cyclo-C₃₋₁₂alkoxy, aryloxy, heteroaryloxy, aryl-C₁₋₆alkoxy, heteroaryl-C₁₋₆alkoxy, amino-C₁-₆alkyl, hydroxy-C₁₋₆alkyl, carboxy-C₁₋₆alkyl, carbamoyl-C₁₋₆alkyl, C₁₋₆alkoxy-C₁₋₆alkyl, hydroxy-C₁₋₆alkoxy, C₁₋₆alkoxy-C₁₋₆alkoxy, carboxy-C₁₋₆alkoxy, C₁₋₆alkoxycarbonyl, C₁₋₆alkylamino, cyclo-C₃₋₁₂alkylamino, arylamino, heteroarylamino, aryl-C₁₋₆alkylamino, heteroaryl-C₁₋₆alkylamino, hydroxy-C₁₋₆alkylamino, carboxy-C₁₋₆alkylamino; C₁₋₆alkylamino-C₁₋₆alkyl, di-(C₁₋₆alkyl)amino, acylamino, di-(C₁₋₆alkyl)amino-C₁₋₆alkyl, carboxy-C₁₋₆alkylamino-C₁₋₆alkyl, C₁₋₆alkylamíno-C₁₋₆alkoxy, di-(C₁₋₆alkyl)amino-C₁₋₆alkoxy, carboxy-C₁₋₆alkylamino-C₁₋₆alkoxy, C₁₋₆alkylsulfonylamino, arylsulfonylamino, C₁₋₆alkylsulfonylamino-C₁₋₆alkyl, C₁₋₆alkyl-aminosulfonyl, di-(C₁₋₆alkyl)aminosulfonyl, arylaminosulfonyl, heteroarylaminosulfonyl, C₁₋₆alkylaminocarbonyl-C₁₋₆alkyl, cyclo-C₃₋₁₂alkylaminocarbonyl-C₁₋₆alkyl, arylaminocarbonyl-C₁₋₆alkyl, heteroarylaminocarbonyl-C₁₋₆alkyl, aryl-C₁₋₆alkylaminocarbonyl-C₁₋₆alkyl, heteroaryl-C₁₋₆alkylaminocarbonyl-C₁₋₆alkyl, hydroxy-C₁₋₆alkylaminocarbonyl-C₁₋₆alkyl, carboxy-C₁₋₆alkylaminocarbonyl-C₁₋₆ alkyl, di-(C₁₋₆alkyl)aminocarbonyl-C₁₋₆alkyl, aryl(C₁₋₆alkyl)aminocarbonyl-C₁₋₆alkyl, and heteroaryl(C₁₋₆alkyl)aminocarbonyl-C₁₋₆alkyl;
the term "heteroaryl" represents an aromatic 5-6 membered ring comprising one to four heteroatoms selected from oxygen, sulfur and nitrogen, or a bicyclic group containing a 5-6 membered ring comprising one to four heteroatoms selected from oxygen, sulfur and nitrogen fused with a benzene ring or with a 5-6 membered ring comprising one to four heteroatoms selected from oxygen, sulfur and nitrogen,
wherein the heteroaryl is optionally substituted by one or more substituents selected independently from halogen, amino, hydroxy, nitro, cyano, COOH, trifluoromethyl, C₁₋₆alkyl, C₂₋₆alkenyl, G₂₋₆alkynyl, heteroaryl, C₁₋₆alkoxy, difluoromethoxy, trifluoromethoxy, cyclo-C₃₋₁₂alkoxy, aryloxy, heteroaryloxy, aryl-C₁₋₆alkoxy, heteroaryl-C₁₋₆alkoxy, amino-C₁₋₆alkyl, hydroxy-C₁₋₆alkyl, carboxy-C₁₋₆alkyl, carbamoyl-C₁₋₆alkyl, C₁₋₆alkoxy-C₁₋₆alkyl, hydroxy-C₁₋₆alkoxy, C₁₋₆alkoxy-C₁₋₆alkoxy, carboxy-C₁₋₆alkoxy, C₁₋₆alkoxycarbonyl, C₁₋₆alkylamino, cyclo-C₃₋₁₂alkylamino, arylamino, heteroarylamino, aryl-C₁₋₆alkylamino, heteroaryl-C₁₋₆alkylamino, hydroxy-C₁₋₆alkylamino, carboxy-C₁₋₆alkylamino, C₁₋₆alkylamino-C₁₋₆alkyl, di-(C₁₋₆alkyl)amino, acylamino, di-(C₁₋₆alkyl)amino-C₁₋₆alkyl, carboxy-C₁₋₆alkylamino-C₁₋₆alkyl, C₁₋₆alkylamino-C₁₋₆alkoxy, di-(C₁₋₆alkyl)amino-C₁₋₆alkoxy, carboxy-C₁₋₆akylamino-C₁₋₆alkoxy, C₁₋₆alkylsulfonylamino, arylsulfonylamino, C₁₋₆alkylsulfonylamino-C₁₋₆alkyl, C₁₋₆alkyl-aminosulfonyl, di-(C₁₋₆alkyl)aminosulfonyl, arylaminosulfonyl, heteroarylaminosulfonyl, C₁₋₆alkylaminocarboriyl-C₁₋₆alkyl, cydo-C_{3- 12}alkylaminocarbonyl-C₁₋₆alkyl, arylaminocarbonyl-C₁₋₆alkyl, heteroarylaminocarbonyl-C₁₋₆alkyl, aryl-C₁₋₆alkylaminocarbonyl-C₁₋₆alkyl, heteroaryl-C₁₋₆alkylaminocarbonyl-C₁₋₆alkyl, hydroxy-C₁₋₆alkylaminocarbonyl-C₁₋₆alkyl, carboxy-C₁₋. ₆alkylaminocarbonyl-C₁₋₆alkyl di-(C₁₋₆alkyl)aminocarbonyl-C₁₋₆alkyl, aryl(C₁₋₆alkyl)aminocarbonyl-C₁₋₆alkyl, heteroaryl(C₁₋₆alkyl)aminocarbonyl-C₁₋₆alkyl;
its optical isomers, polymorphs and pharmaceutically-acceptable acid and base addition salts and hydrates and solvates thereof;
it being understood that:
if R² and R³ together represent -(CH2)ₙ-, then R¹ does not represent aryl-C₁₋₆alkyl or heteroaryl-C₁₋₆alkyl.

2. The compound as claimed in Claim 1, wherein R¹ represents hydrogen, COOH, aryl, arylaminocarbonyl, heteroarylaminocarbonyl, arylC₁₋₆alkylamino, heteroarylC₁₋₆alkylamino, acylamino, arylsulfonylamino, C₁₋₆alkoxyoarbonyl, aryl-C₁₋₆alkylaminocarbonyl, arylC₂₋₆alkenyl, arylC₁₋₆alkoxycarbonyl, heteroarylC₁₋₆alkoxycarbonyl, aryl(C₁₋₆alkyl)aminocarbonyl, heteroaryl(C₁₋₆alkyl)aminocarbonyl, aryl-heterocyclyl-C₁₋₆alkyl, or heteroaryl-heterocyclyl-C₁₋₆alkyl.

3. The compound as claimed in Claim 2, wherein R¹ represents arylcarbonylamino or heteroarylcarbonylamino.

4. The compound as claimed in Claim 2, wherein R¹ represents arylaminocarbonyl, heteroarylaminocarbonyl, arylC₁₋₆alkylamino, heteroarylC₁₋₆alkylamino, arylcarbonylamino, heteroarylcarbonylamino, arylsulfonylamino, arylC₁₋₆alkoxycarbonyl, heteroarylC₁₋₆alkoxycarbonyl, aryl(C₁₋₆alkyl)aminocarbonyl, heteroaryl(C₁₋₆alkyl)aminocarbonyl, aryl-heterocyclyl-C₁₋₆alkyl, or heteroaryl-heterocyclyl-C₁₋₆alkyl, wherein the aryl or heteroaryl moeity is optionally substituted by one or more substituents selected from halogen, hydroxy, trifluoromethyl, C₁₋₆alkyl, and C₁₋₆alkoxy.

5. The compound as claimed in Claim 4, wherein R¹ represents aryl-C₁₋₆alkylamino or heteroaryl-C₁₋₆alkylamino wherein the C₁₋₆alkyl moiety is substituted by 1 or more carboxy groups.

6. The compound as claimed in any of Claims 1 to 5, wherein R² and R³ together represent -(CH₂)ₙ- wherein n is 3, 4, or 5 or R² and R³ together with the C=C bond to which they are attached represent a phenyl ring.

7. The compound as claimed in Claim 6, wherein R² and R³ together represent -(CH₂)ₙ- wherein n is 4.

8. The compound as claimed in Claim 1, which is selected from:
5,6,7,8-Tetrahydro-1H,4H-pyrazolo[5,1-b]quinazoline-2,9-dione,
3-Phenyl-5,6,7,8-tetrahydro-1H,4H-pyrazolo[5,1-b]quinazoline-2,9-dione,
2,9-Dioxo-1,2,4,5,6,7,8,9-octahydro-pyrazolo[5,1-b]quinazoline-3-carboxylic acid phenylamide,
N-(2,9-Dioxo-1,2,4,5,6,7,8,9-octahydro-pyrazolo[5,1-b]quinazolin-3-yl)benzamide, Ethyl 2,9-dioxo-1,2,4,5,6,7,8,9-octahydro-pyrazolo[5,1-b]quinazoline-3-carboxylate,
2,9-Dioxo-1,2,4,5,6,7,8,9-octahydro-pyrazolo[5,1-b]quinazoline-3-carboxylic acid (4-chloro-phenyl)-amide,
2,9-Dioxo-1,2,4,5,6,7,8,9-octahydro-pyrazolo[5,1-b]quinazoline-3-carboxylic acid (3-ch)oro-phenyl)-amide,
2,9-Dioxo-1,2,4,5,6,7,8,9-octahydro-pyrazolo[5,1-b]quinazoline-3-carboxylic acid (2-chloro-phenyl)-amide,
2,9-Dioxo-1,2,4,5,6,7,8,9-octahydro-pyrazolo[5,1-b]quinazoline-3-carboxylic acid benzylamide,
3-((E)-Styryl)-5,6,7,8-tetrahydro-1H,4H-pyrazolo[5,1-b]quinazoline-2,9-dione,
3-[(E)-2-(4-Chloro-phenyl)-vinyl]-5,6,7,8-tetrahydro-1H,4H-pyrazolo[5,1-b]quinazoline-2,9-dione,
3-[(E)-2-(3-Chloro-phenyl)-vinyl]-5,6,7,8-tetrahydro-1H,4H-pyrazolo[5,1-b]quinazoline-2,9-dione,
3-[(2,9-Dioxo-1,2,4,5,6,7,8,9-octahydro-pyrazolo[5,1-b]quinazoline-3-carbonyl)-amino]-benzoic acid,
Benzyl 2,9-dioxo-1,2,4,5,6,7,8,9-octahydro-pyrazolo[5,1-b]quinazoline-3-carboxylate,
2,9-Dioxo-1,2,4,5,6,7,8,9-octahydro-pyrazolo[5,1-b]quinazoline-3-carboxylic acid (4-methoxy-phenyl)-amide,
2,9-Dioxo-1,2,4,5,6,7,8,9-octahydro-pyrazolo[5,1-b]quinazoline-3-carboxylic acid pyridin-3-ylamide,
N-(2,9-Dioxo-1,2,4,9-tetrahydro-pyrazolo[5,1-b]quinazolin-3-yl)-benzamide,
N-(2,9-Dioxo-1,2,4,9-tetrahydro-pyrazolo[5,1-b]quinazolin-3-yl)4-methoxybenzamide,
N-(2,9-Dioxo-1,2,4,9-tetrahydro-pyrazolo[5,1-b]quinazolin-3-yl)-2-fluoro-benzamide,
N-(2,9-Dioxo-1,2,4,9-tetrahydro-pyrazolo[5,1-b]quinazolin-3-yl)-3-chloro-benzamide,
2,9-Dioxo-1,2,4,5,6,7,8,9-octahydro-pyrazolo[5,1-b]quinazoline-3-carboxylic acid pyridin-2-ylamide,
2,9-Dioxo-1,2,4,5,6,7,8,9-octahydro-pyrazolo[5,1-b]quinazoline-3-carboxylic acid pyrid)n-4-ylamide,
2,9-Dioxo-1,2,4,5,6,7,8,9-octahydro-pyrazolo[5,1-b]quinazoline-3-carboxylic acid furan-3-ylamide,
2,9-Dioxo-1,2,4,5,6,7,8,9-octahydro-pyrazolo[5,1-b]quinazoline-3-carboxylic acid (1H-pyrrol-2-yl)-amide,
2,9-Dioxo-1,2,4,5,6,7,8,9-octahydro-pyrazolo[5,1-b]quinazoline-3-carboxylic acid (3-hydroxy-phenyl)-amide,
2,9-Dioxo-1,2,4,5,6,7,8,9-octahydro-pyrazolo[5,1-b]quinazoline-3-carboxylic acid (4-hydroxy-phenyl)-amide
2,9-Dioxo-1,2,4,5,6,7,8,9-octahydro-pyrazolo[5,1-b]quinazoline-3-carboxylic acid (2-fluoro-phenyl)-amide,
2,9-Dioxo-1,2,4,5,6,7,8,9-octahydro-pyrazolo[5,1-b]quinazoline-3-carboxylic acid (2-hydroxy-phenyl)-amide,
N-(2,9-Dioxo-1,2,4,5,6,7,8,9-octahydro-pyrazolo[5,1-b]quinazolin-3-yl)-2-fluoro-benzamide,
N-(2,9-Dioxo-1,2,4,5,6,7,8,9-octahydro-pyrazolo[5,1-b]quinazolin-3-yl)-2-hydroxy-benzamide,
3-[(E)-2-(2-Fluoro-phenyl)-vinyl]-5,6,7,8-tetrahydro-1H,4H-pyrazolo[5,1-b]quinazoline-2,9-dione,
2,9-Dioxo-1,2,4,9-tetrahydro-pyrazolo[5,1-b]quinazoline-3-carboxylic acid, 2,9-Dioxo-1,2,4,9-tetrahydro-pyrazolo[5,1-b]quinazoline-3-carboxylic acid phenylemide,
2,9-Dioxo-1,2,4,9-tetrahydro-pyrazolo[5,1-b]quinazoline-3-carboxylic acid (4-methoxy-phenyl)-amide,
2,9-Dioxo-1,2,4,9-tetrahydro-pyrazolo[5,1-b]quinazoline-3-carboxylic acid (2-fluoro-phenyl)-amide,
2,9-Dioxo-1,2,4,9-tetrahydro-pyrazolo[5,1-b]quinazoline-3-carboxylic acid (3-chloro-phenylyamide,
3-(4-Fluoro-phenylamino)-5,6,7,8-tetrahydro-1H,4H-pyrazolo[5,1-b]quinazoline-2,9-dione,
3-(4-Fluoro-phenylamino)-1H,4H-pyrazolo[5,1-b]quinazoline-2,9-dione,
N-(2,9-Dioxo-1,2,4,5,6,7,8,9- octahydro-pyrazolo[5,1-b]quinazolin-3-yl)-4-methyl-benzenesulfonamide,
N-(2,9-Dioxo-1,2,4,9-tetrahydro-pyrazolo[5,1-b]quinazolin-3-yl)-4-methyl-benzenesulfonamide,
2-(2,9-Dioxo-1,2,4,5,6,7,8,9-octahydro-pyrazolo[5,1-b]quinazolin-3-ylamino)3-phenyl-propionic acid,
2-(2,9-Dioxo-1,2,4,9-tetrahydro-pyrazolo[5,1-b]quinazolin-3-ylamino)-3-phenylpropionic acid,
and optical isomers, polymorphs, and pharmaceutically-acceptable acid and base addition salts, hydrates, and solvates thereof.

9. A pharmaceutical composition comprising as active ingredient a compound as claimed in any preceding claim, optionally together with one or more pharmaceutically acceptable excipients.

10. A compound as claimed in any of Claims 1 to 8 for use in the treatment or prevention of NMDA excitotoxicity or malfunctioning glutamatergic neruotransmission.

11. A compound as claimed in any of Claims 1 to 8 for the prevention and/or treatment of a condition selected from: pain, including acute pain, chronic pain, allodynia, hyperalgesia, visceral pain, phantom pain, post-operative pain, neuropathic pain, peripheral neuropathy including, for example peripheral neuropathy induced by nociception, inflammation, ischemia, viral infection (HZV), traumatic and other mechanical nerve injury, cancer, diabetes mellitus, HIV infection, fibromyalgia, trigeminus neuralgia, inflammatory bowel diseases (IBO), irritative bowel syndrome (IBS), arthritis including rheumatoid arthritis, osteoarthritis (degenerative joint disease), multiple sclerosis (MS) and gout (metabolic arthritis); acute insults, including cerebral ischemia, cerebral infarct, brain oedema, anoxia, inner ear insult, inner ear insult in tinnitus, head or brain or spinal cord trauma, head or brain or spinal cord injuries, trauma, sound- or drug-induced inner ear insult, ischaemia resulting from cardiac arrest or stroke or bypass operations or transplants, acute pain, hypoxia, perinatal hypoxia, and ischaemia; chronic insults, such as neurodegenerative disorders, including Morbus Huntington, Alzheimer's disease Creutzfeld-Jakob's syndrome/disease, bovine spongiform encephalopathy (BSE) prion related infections, diseases involving mitochondrial dysfunction, diseases involving β-amyloid and/or tauopathy, Down's syndrome, motor neuron diseases, amyotrophic lateral sclerosis (ALS), olivoponto-cerebellar atrophy, Parkinson's disease, Neuronal Ceroid Lipofuscinosis, AIDS dementia complex, AIDS-related dementia, dementia related to HIV infections, HIV-1 encephalopathy, AIDS encephalopathy, Korsakoff syndrome, vascular dementia, and corticobasal degeneration; neurological disorders, including tinnitus, hearing loss, sound- or drug-induced tinnitus, haloperidol-induced dyskinesias, dopaminomimetic-induced dyskinesias, chorea, Huntington's chorea, athetosis, dystonia, stereotypy, ballism, tardive dyskinesias, tic disorder, spasmodic torticollis, blepharospasm, focal and generalized dystonia, nystagmus, Parkinson's dementia, mild cognitive impairment, cognitive deficits in various forms of mild cognitive impairment, cognitive deficits in various forms of dementia, dementia pugilistica, vascular and frontal lobe dementia, cognitive impairment, learning impairment, L-dopa-induced dykinesias, L-dopa-induced dykinesias in Parkinson's disease therapy, dyskinesias, dyskinesia in Huntington's disease, drug induced dyskinesias, neuroleptic-induced dyskinesias, neurodegenerative cerebellar ataxias, centrally induced neuropathic pain, convulsions, epileptic convulsions, epilepsy, temporal lobe epilepsy, myoclonic epilepsy, tremor, dementia in Alzheimer's disease, dementia in Korsakoff syndrome, dementia, hereditary cerebellar ataxias, sleep disorders, movement disorders, essential tremor, muscle spasms, and spasticity; psychological/psychiatric disorders, including generalized anxiety disorder, obsessive-compulsive disorder, panic disorder, posttraumatic stress disorder, social phobia, phobic disorders, substance-induced anxiety disorder, delusional disorder, schizoaffective disorder, schizophreniform disorder, substance-induced psychotic disorder, delirium, post-operative cognitive deficit (POCD), cognitive impairment, learning impairment, anxiety disorders, panic disorders, anxiety and panic disorders, social anxiety disorder (SAD), attention deficit hyperactivity disorder (ADHD), attention deficit syndrome (ADS), dementia, posttraumatic stress disorder (PTSD), schizophrenia, positive or cognitive or negative symptoms of schizophrenia, major depressive disorder, major depression, depression, bipolar manic-depressive disorder, sleep disorders, agoraphobia, bulimia nervosa, eating disorders, obesity, obesity-related disorders, obesity abuse, food addiction, binge eating disorders, and hyperactivity in children; drug/alcohol abuse, including craving (e.g., for drugs of abuse), abuse, addiction, nicotine addiction, nicotine abuse, alcohol addiction, alcohol abuse, opiate addiction, opiate abuse, cocaine addiction, cocaine abuse, amphetamine addiction, and amphetamine abuse; skin diseases, including atopic dermatitis, itching, skin lesions induced by severe itching or atopic dermatitis, systemic sclerosis, pruritic conditions, and pruritis; diseases of the gastro-intestinal tract and metabolic diseases, including diarrhoea, hepatic encephalopathy, hypoglycaemia, gastroesophageal reflux disease (GERD), gastrointestinal dysfunction, lower esophageal sphincter (LES) disease, functional gastrointestinal disorders, dyspepsia, vomiting, urinary incontinence, and regurgitation; diseases of the immune system, including Sjogren's syndrome, systemic lupus erythematosus, and multiple sclerosis (MS); eye diseases, including eye injuries, eye diseases, eye disorders, glaucoma, retinopathy, and macular degeneration; diseases of the respiratory tract, including respiratory tract infection, chronic laryngitis, asthma, reflux-related asthma, and lung disease; migraine; autism; restless leg syndrome (RLS); Tourette syndrome; micturition disorders; neuromuscular disorder in the lower urinary tract; and drug tolerance to opioids.

12. A compound as claimed in Claim 11, wherein the condition is selected from: pain, acute pain, chronic pain, allodynia, hyperalgesia, visceral pain, phantom pain, post-operative pain, neuropathic pain, peripheral neuropathy including, for example peripheral neuropathy induced by nociception, inflammation, ischemia, viral infection (HZV), traumatic and other mechanical nerve injury, cancer, diabetes mellitus, HIV infection, fibromyalgia, trigeminus neuralgia, inflammatory bowel diseases (IBD), irritative bowel syndrome (IBS), arthritis including rheumatoid arthritis, osteoarthritis (degenerative joint disease), multiple sclerosis (MS) and gout (metabolic arthritis).

13. Use of a compound as claimed in any of Claims 1 to 8 for the manufacture of a medicament for the prevention and/or treatment of a condition selected from: pain, including acute pain, chronic pain, allodynia, hyperalgesia, visceral pain, phantom pain, post-operative pain, neuropathic pain, peripheral neuropathy including, for example peripheral neuropathy induced by nociception, inflammation, ischemia, viral infection (HZV), traumatic and other mechanical nerve injury, cancer, diabetes mellitus, HIV infection, fibromyalgia, trigeminus neuralgia, inflammatory bowel diseases (IBD), irritative bowel syndrome (IBS), arthritis including rheumatoid arthritis, osteoarthritis (degenerative joint disease), multiple sclerosis (MS) and gout (metabolic arthritis); acute insults, including cerebral ischemia, cerebral infarct, brain oedema, anoxia, inner ear insult, inner ear insult in tinnitus, head or brain or spinal cord trauma, head or brain or spinal cord injuries, trauma, sound- or drug-induced inner ear insult, ischaemia resulting from cardiac arrest or stroke or bypass operations or transplants, acute pain, hypoxia, perinatal hypoxia, and ischaemia; chronic insults, such as neurodegenerative disorders, including Morbus Huntington, Alzheimer's disease Creutzfeld-Jakob's syndrome/disease, bovine spongiform encephalopathy (BSE) prion related infections, diseases involving mitochondrial dysfunction, diseases involving β-amyloid and/or tauopathy, Down's syndrome, motor neuron diseases, amyotrophic lateral sclerosis (ALS), olivoponto-cerebellar atrophy, Parkinson's disease, Neuronal Ceroid Lipofuscinosis, AIDS dementia complex, AIDS-related dementia, dementia related to HIV infections, HIV-1 encephalopathy, AIDS encephalopathy, Korsakoff syndrome, vascular dementia, and corticobasal degeneration; neurological disorders, including tinnitus, hearing loss, sound- or drug-induced tinnitus, haloperidol-induced dyskinesias, dopaminomimetic-induced dyskinesias, chorea, Huntington's chorea, athetosis, dystonia, stereotypy, ballism, tardive dyskinesias, tic disorder, spasmodic torticollis, blepharospasm, focal and generalized dystonia, nystagmus, Parkinson's dementia, mild cognitive impairment, cognitive deficits in various forms of mild cognitive impairment, cognitive deficits in various forms of dementia, dementia pugilistica, vascular and frontal lobe dementia, cognitive impairment, learning impairment, L-dopa-induced dykinesias, L-dopa-induced dykinesias in Parkinson's disease therapy, dyskinesia, dyskinesia in Huntington's disease, drug induced dyskinesias, neuroleptic-induced dyskinesias, neurodegenerative cerebellar ataxias, centrally induced neuropathic pain, convulsions, epileptic convulsions, epilepsy, temporal lobe epilepsy, myoclonic epilepsy, tremor, dementia in Alzheimer's disease, dementia in Korsakoff syndrome, dementia, hereditary cerebellar ataxias, sleep disorders, movement disorders, essential tremor, muscle spasms, and spasticity; psychological/psychiatric disorders, including generalized anxiety disorder, obsessive-compulsive disorder, panic disorder, posttraumatic stress disorder, social phobia, phobic disorders, substance-induced anxiety disorder, delusional disorder, schizoaffective disorder, schizophreniform disorder, substance-induced psychotic disorder, delirium, post-operative cognitive deficit (POCD), cognitive impairment, learning impairment, anxiety disorders, panic disorders, anxiety and panic disorders, social anxiety disorder (SAD), attention deficit hyperactivity disorder (ADHD), attention deficit syndrome (ADS), dementia, posttraumatic stress disorder (PTSD), schizophrenia, positive or cognitive or negative symptoms of schizophrenia, major depressive disorder, major depression, depression, bipolar manic-depressive disorder, sleep disorders, agoraphobia, bulimia nervosa, eating disorders, obesity, obesity-related disorders, obesity abuse, food addiction, binge eating disorders, and hyperactivity in children; drug/alcohol abuse, including craving (e.g., for drugs of abuse), abuse, addiction, nicotine addiction, nicotine abuse, alcohol addiction, alcohol abuse, opiate addiction, opiate abuse, cocaine addiction, cocaine abuse, amphetamine addiction, and amphetamine abuse; skin diseases, including atopic dermatitis, itching, skin lesions induced by severe itching or atopic dermatitis, systemic sclerosis, pruritic conditions, and pruritis; diseases of the gastro-intestinal tract and metabolic diseases, including diarrhoea, hepatic encephalopathy, hypoglycaemia, gastroesophageal reflux disease (GERD), gastrointestinal dysfunction, lower esophageal sphincter (LES) disease, functional gastrointestinal disorders, dyspepsia, vomiting, urinary incontinence, and regurgitation; diseases of the immune system, including Sjogren's syndrome, systemic lupus erythematosus, and multiple sclerosis (MS); eye diseases, including eye injuries, eye diseases, eye disorders, glaucoma, retinopathy, and macular degeneration; diseases of the respiratory tract, including respiratory tract infection, chronic laryngitis, asthma, reflux-related asthma, and lung disease; migraine; autism; restless leg syndrome (RLS); Tourette syndrome; micturition disorders; neuromuscular disorder in the lower urinary tract; and drug tolerance to opioids.

14. Use as claimed in Claim 13, wherein the medicament is manufactured for the prevention and/or treatment of a condition selected from: pain, acute pain, chronic pain, allodynia, hyperalgesia, visceral pain, phantom pain, post-operative pain, neuropathic pain, peripheral neuropathy including, for example peripheral neuropathy induced by nociception, inflammation, ischemia, viral infection (HZV), traumatic and other mechanical nerve injury, cancer, diabetes mellitus, HIV infection, fibromyalgia, trigeminus neuralgia, inflammatory bowel diseases (IBD), irritative bowel syndrome (IBS), arthritis including rheumatoid arthritis, osteoarthritis (degenerative joint disease), multiple sclerosis (MS) and gout (metabolic arthritis).

15. A process for the synthesis of a compound selected from those of Formula I: 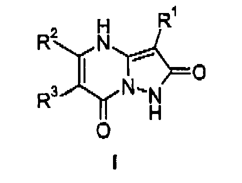 wherein
R¹ represents hydrogen, trifluoromethyl, C₁₋₆alkyl, COOH, C₁₋₆alkoxycarbonyl, hydroxy-C₁₋₆alkoxycarbonyl, aryl-C₁₋₆alkoxycarbonyl, heteroaryl-C₁₋₆alkoxycarbonyl, aryl, heteroaryl, aryl-C₁₋₆alkyl, heteroaryl-C₁₋₆alkyl; cyclo-C₅₋₁₂alkyl-C₁₋₆alkyl, aryl-C₂₋₆alkenyl, heteroaryl-C₂₋₆alkenyl, arylamino, heteroarylamino, aryl-C₁₋₆alkylamino, heteroaryl-C₁₋₆alkylamino, acylamino, arylsulfonylamino, C₁₋₆alkylaminocarbonyl, cyclo-C₃₋₁₂alkylaminocarbonyl, arylaminocarbonyl, heteroarylaminocarbonyl, aryl-C₁₋₆alkylaminocarbonyl, heteroaryl-C₁₋₆alkylaminocarbonyl, hydroxy-C₁₋₆alkylaminocarbonyl, di-(C₁₋₆alkyl)aminocarbonyl, aryl(C₁₋₆alkyl)aminocarbonyl, heteroaryl(C_{1- 6}alkyl)aminocarbonyl, hydroxy-C₁₋₆alkyl, C₁₋₆alkoxy-C₁₋₆alkyl, carboxy-C₁₋₆alkyl, carbamoyl-C₁₋₆alkyl, amino-C₁₋₆alkyl, acylamino-C₁₋₆alkyl, arylamino-C₁₋₆alkyl, aryloxy-C₁₋₆alkyl, C₁₋₆alkylsulfonylamino-C₁₋₆alkyl, arylsulfonylamino-C₁₋₆alkyl, C₁₋₆alkylaminocarbonyl-C₁₋₆alkyl, cyclo-C₃₋₁₂alkylaminocarbonyl-C₁₋₆alkyl, arylaminocarbonyl-C₁₋₆alkyl, heteroarylaminocarbonyl-C₁₋₆alkyl, aryl-C₁₋₆alkylaminocarbonyl-C₁₋₆alkyl, heteroaryl-C₁₋₆alkylaminocarbonyl-C₁₋₆alkyl, hydroxy-C₁₋₆alkylaminocarbonyl-C₁₋₆alkyl, carboxy-C₁₋₆alkylaminocarbonyl-C₁₋₆alkyl, di-(C₁₋₆alkyl)aminocarbonyl-C₁₋₆alkyl, aryl(C₁₋₆alkyl)aminocarbonyl-C₁₋₆alkyl, heteroaryl(C₁₋₆alkyl)aminocarbonyl-C₁₋₆alkyl, aryl-heterocyclyl-C₁₋₆alkyl, or heteroaryl-heterocyclyl-C₁₋₆alkyl; and
R² and R³ together represent -(CH₂)ₙ- wherein n is 3, 4, or 5;
or R² and R³ together with the C=C bond they are attached represent a phenyl ring or a 5-, 6- or 7-membered ring which may be unsaturated, or partly unsaturated, and wherein the ring in addition to nitrogen atom may contain heteroatom selected from sulfur, oxygen and nitrogen and may be substituted by one or more substituents selected from C₁₋₆alkyl and halogen;
wherein
the term "aryl" represents phenyl or naphthyl, or phenyl substituted by one or more substituents selected independently from halogen, amino, hydroxy, nitro, cyano, COOH, trifluoromethyl, C₁₋₆alkyl, C₂₋₆alkenyl, C₂₋₆alkynyl, heteroaryl, C₁₋₆alkoxy, difluoromethoxy, trifluoromethoxy, cyclo-C₃₋₁₂alkoxy, aryloxy, heteroaryloxy, aryl-C₁₋₆alkoxy, heteroaryl-C₁₋₆alkoxy, amino-C₁₋₆alkyl, hydroxy-C₁₋₆alkyl, carboxy-C₁₋₆alkyl, carbamoyl-C₁₋₆alkyl, C₁₋₆alkoxy-C₁₋₆alkyl, hydroxy-C₁₋₆alkoxy, C₁₋₆alkoxy-C₁₋₆alkoxy, carboxy-C₁₋₆alkoxy, C₁₋₆alkoxycarbonyl, C₁₋₆alkylamino, cyclo-C₁₋₁₂alkylamino, arylamino, heteroarylamino, aryl-C₁₋₆alkylamino, heteroaryl-C₁₋₆alkylamino, hydroxy-C₁₋₆alkylamino, carboxy-C₁₋₆alkylamino, C₁₋₆alkylamino-C₁₋₆alkyl, di-(C₁₋₆alkyl)amino, acylamino, di-(C₁₋₆alkyl)amino-C₁₋₆alkyl, carboxy-C₁₋₆alkylamino-C₁₋₆alkyl, C₁₋₆alkylamino-C₁₋₆alkoxy, di-(C₁₋₆alkyl)amino-C₁₋₆alkoxy, carboxy-C₁₋₆alkylamino-C₁₋₆alkoxy, C₁₋₆alkylsulfonylamino, arylsulfonylamino, C₁₋₆alkylsulfonylamino-C₁₋₆alkyl, C₁₋₆alkyl-aminosulfonyl, di-(C₁₋₆alkyl)aminosulfonyl, arylaminosulfonyl, heteroarylaminosulfonyl, C₁₋₆alkylaminocarbonyl-C₁₋₆alkyl, cyclo-C₃₋₁₂alkylaminocarbonyl-C₁₋₆alkyl, arylaminocarbonyl-C₁₋₆alkyl, heteroarylaminocarbonyl-C₁₋₆alkyl, aryl-C₁₋₆alkylaminocarbonyl-C₁₋₆alkyl, heteroaryl-C₁₋₆alkylamihocarbonyl-C₁₋₆alkyl, hydroxy-C₁₋₆alkylaminocarbonyl-C_{1- 6}alkyl, carboxy- C₁₋₆alkylaminocarbonyl-C₁₋₆alkyl, di-(C₁₋₆alkyl)aminocarbonyl-C₁₋₆alkyl, aryl(C₁₋₆alkyl)aminocarbonyl-C₁₋₆alkyl, and heteroaryl(C₁₋₆alkyl)aminocarbonyl-C₁₋₆alkyl;
the term "heteroaryl" represents an aromatic 5-6 membered ring comprising one to four heteroatoms selected from oxygen, sulfur and nitrogen, or a bicyclic group containing a 5-6 membered ring comprising one to four heteroatoms selected from oxygen, sulfur and nitrogen fused with a benzene ring or with a 5-6 membered ring comprising one to four heteroatoms selected from oxygen, sulfur and nitrogen,
wherein the heteroaryl is optionally substituted by one or more substituents selected independently from halogen, amino, hydroxy, nitro, cyano, COOH, trifluoromethyl, C₁₋₆alkyl, C₂₋₆alkenyl, C₂₋₆alkynyl, heteroaryl, C₁₋₆alkoxy, difluoromethoxy, trifluoromethoxy, cyclo-C₃₋₁₂alkoxy, aryloxy, heteroaryloxy, aryl-C₁₋₆alkoxy, heteroaryl-C₁₋₆alkoxy, amino-C₁₋₆alkyl, hydroxy-C₁₋₆alkyl, carboxy-C₁₋₆alkyl, carbamoyl-C₁₋₆alkyl, C₁₋₆alkoxy-C₁₋₆alkyl, hydroxy-C₁₋₆alkoxy, C₁₋₆alkoxy-C₁₋₆alkoxy, carboxy-C₁₋₆alkoxy, C₁₋₆alkoxycarbonyl, C₁₋₆alkylamino, cyclo-C₃₋₁₂alkylamino, arylamino, heteroarylamino, aryl-C₁₋₆alkylamino, heteroaryl-C₁₋₆alkylamino, hydroxy-C₁₋₆alkylamino, carboxy-C₁₋₆alkylamino, C₁₋₆alkylamino-C₁₋₆alkyl, di-(C₁₋₆alkyl)amino, acylamino, di-(C₁₋₆alkyl)amino-C₁₋₆alkyl, carboxy-C₁₋₆alkylamino-C₁₋₆alkyl, C₁₋₆alkylamino-C₁₋₆alkoxy, di-(C₁₋₆alkyl)amino-C₁₋₆alkoxy, carboxy-C₁₋₆alkylamino-C₁₋₆alkoxy, C₁₋₆alkylsulfonylamino, arylsulfonylamino, C₁₋₆alkylsulfonylamino-C₁₋₆alkyl, C₁₋₆alkyl-aminosulfonyl, di-(C₁₋₆alkyl)aminosulfonyl, arylaminosulfonyl, heteroarylaminosulfonyl, C₁₋₆alkylaminocarbonyl-C₁₋₆alkyl, cyclo-C₃₋₁₂alkylaminocarbonyl-C₁₋₆alkyl, arylaminocarbonyl-C₁₋₆alkyl, heteroarylaminocarbonyl-C₁₋₆alkyl, aryl-C₁₋₆alkylaminocarbonyl-C₁₋₆alkyl, heteroaryl-C₁₋₆alkylaminocarbonyl-C₁₋₆alkyl, hydroxy-C₁₋₆alkylaminocarbonyl-C₁₋₆alkyl, carboxy-C₁₋₆alkylaminocarbonyl-C₁₋₆alkyl di-(C₁₋₆alkyl)aminocarbonyl-C₁₋₆alkyl, aryl(C₁-₆alkyl)aminocarbonyl-C₁₋₆alkyl, heteroaryl(C₁₋₆alkyl)aminocarbonyl-C₁₋₆alkyl;
its optical isomers polymorphs , analogs, derivatives, prodrugs, and pharmaceutically-acceptable acid and base addition salts and hydrates and solvates thereof;
it being understood that:
if R² and R³ together represent - (CH2)ₙ-, then R¹ does not represent aryl-C₁₋₆alkyl or heteroaryl-C₁₋₆alkyl, comprising reaction of a compound of Formula II: 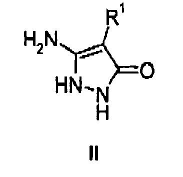
with a compound of Formula III: 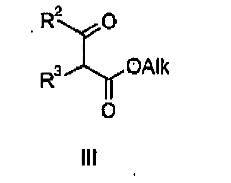 wherein Alk represents an alkyl group (e.g., Me, Et), optionally in the presence of base in an appropriate solvent (e.g., sodium hydroxide or potassium hydroxide in aqueous alcohol, sodium ethylate in ethanol, or piperdine in DMF) to yield a compound of Formula I, which may be converted, if desired, into an optical isomer, polymorph, analog, derivative, prodrug, pharmaceutically-acceptable salt, hydrate or solvate.
